# EUROPEAN PATENT APPLICATION

(11) **EP 1 574 504 A1**
(43) Date of publication of application: **14.09.2005**
(21) Application number: 03777317.3
(22) Date of filing: 05.12.2003
(51) Int. Cl.: C07D 265/36, C07D 413/12, A61K 31/538, A61P 7/02, A61P 9/00, A61P 9/10

(54) **BENZOMORPHOLINE DERIVATIVES**

(30) Priority: 06.12.2002 JP 2002355544
(71) Applicant: TORAY INDUSTRIES, INC., Tokyo 103-8666 (JP)
(72) Inventor: OHNO, Michihiro, Yokohama-shi, Kanagawa 245-0061 (JP); HAYASHI, Ryoji, Fujisawa-shi, Kanagawa 251-0033 (JP); ISOGAYA, Masafumi, Yokohama-shi, Kanagawa 245-0016 (JP); UEDA, Hiroshi, Kamakura-shi, Kanagawa 248-0031 (JP)
(74) Representative: Oser, Andreas
(86) International application number: PCT/JP2003/015631
(87) International publication number: WO 2004/052871

(57) **Abstract**

This invention relates to benzomorpholine derivatives typified by a compound represented by general formula (VIII) 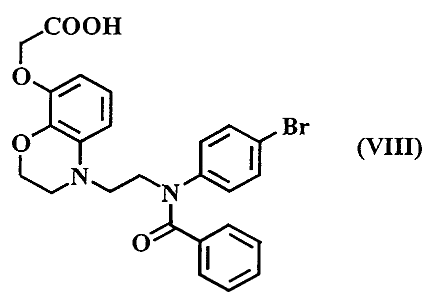 or pharmaceutically acceptable salts thereof and platelet aggregation inhibitors comprising the same. The compounds of the present invention have a strong inhibitory effect of platelet aggregation, so that they are effective for treating and preventing diseases in which thrombus is involved.

## Description

### Technical Field

The present invention relates to benzomorpholine derivatives having a strong inhibitory effect of platelet aggregation and a drug comprising the same as an active ingredient.

### Background Art

When a blood vessel is damaged and begins to bleed, coagulation occurs to cover the wound of the blood vessel and to stop bleeding. Hemostasis is a function necessary for continuation of life and coagulation is an important biological defense reaction. Coagulation occurs first via platelet aggregation. However, coagulation inside the blood vessel, namely in the form of thrombus, inhibits blood circulation when it becomes excessive, thereby causing the onset of myocardial infarction, cerebral infarction, and many other thrombotic diseases. The thrombotic diseases are today's leading causes of death, along with cancer. The prevention thereof and treatment therefor are strongly desired. To treat and prevent such thrombotic diseases, drugs that strongly inhibit thrombogenesis (specifically, platelet aggregation) are required.

Various compounds have been developed to inhibit platelet aggregation. In particular, aspirin, thienopyridine derivatives (ticlopidine and clopidogrel), and the like are known. However, the platelet aggregation inhibitory effect of aspirin is weak and insufficient. Furthermore, there are concerns about the side effects of aspirin, such as gastritis, peptic ulcer, and the like.

Furthermore, regarding ticlopidine, which is a thienopyridine derivative, side effects such as thrombotic thrombocytopenic purpura and hepatopathy are known. Hence, development of a safer and more effective novel platelet aggregation inhibitor is also currently required.

As a result of various studies to create novel compounds that strongly inhibit platelet aggregation, the present inventors have discovered that benzomorpholine derivatives having a side chain with an amide structure have a strong inhibitory effect of platelet aggregation.

Patent document 1 already discloses benzomorpholine derivatives having an inhibitory effect of platelet aggregation. However, the platelet aggregation inhibitory effect thereof is weak and the document does not disclose specifically compounds having an amide structure, which is a characteristic of the compounds of the present invention.

### Patent document 1

### International Publication No. 00/07992 pamphlet

Moreover, patent document 2 discloses azabicyclic compounds containing benzomorpholine rings. However, patent document 2 never discloses benzomorpholine derivatives, which are disclosed in the present invention. Furthermore, the compounds in patent document 2 are compounds that inhibit the binding between VCAM-1 and VLA4. Patent document 2 does not describe an anti-platelet effect at all.

### Patent document 2

### International Publication No. 00/39103 pamphlet

### Disclosure of the Invention

An object of the present invention is to provide novel compounds having an inhibitory effect of platelet aggregation.

That is, the present invention encompasses the following invention.
(A) A benzomorpholine derivative or pharmaceutically acceptable salt thereof represented by formula I, 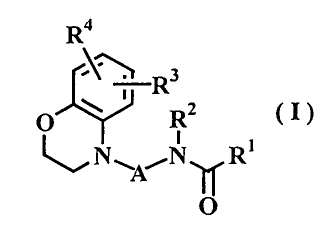 wherein
   A is C₂₋₄ alkylene, C₂₋₄ alkenylene, or C₂₋₄ alkynylene,
   R¹ is:
   (1) unsubstituted aryl or heteroaryl, or aryl or heteroaryl substituted with one or a plurality of substituents independently selected from the following group,
      a) C₁₋₅ alkyl, b) C₁₋₅ alkoxy c) C₃₋₈ cycloalkyl, d) C₁₋₅ haloalkyl, e) phenyl, f) phenoxy, g) hydroxyl, h) C₁₋₅ hydroxyalkyl, i) C₁₋₅ haloalkyloxy, j) mercapto, k) C₁₋₅ alkylthio, I) C₁₋₅ haloalkylthio, m) halogen, n) cyano, o) nitro, p) amino, q) C₁₋₅ alkylamino, r) C₂₋₁₀ dialkylamino, s) acyl, t) carboxyl, u) C₂₋₆ alkyloxycarbonyl, v) mesyl, w) trifluoromethanesulfonyl, and x) tosyl; or
   (2) unsubstituted C₁₋₅ alkyl, C₃₋₈ cycloalkyl, C₂₋₁₀ alkenyl, C₄₋₁₀ cycloalkenyl, or C₂₋₁₀ alkynyl, or C₁₋₅ alkyl, C₃₋₈ cycloalkyl, C₂₋₁₀ alkenyl, C₄₋₁₀ cycloalkenyl, or C₂₋₁₀ alkynyl substituted with one or a plurality of substituents independently selected from the following group,
      a) phenyl, b) hydroxyl, c) C₁₋₅ alkyl, d) C₃₋₈ cycloalkyl, e) C₁₋₅ haloalkyl, and f) halogen;
      R² is unsubstituted aryl or heteroaryl, or aryl or heteroaryl substituted with one or a plurality of substituents independently selected from the following group,
      a) C₁₋₅ alkyl, b) C₁₋₅ alkoxy, c) C₃₋₈ cycloalkyl, d) C₁₋₅ haloalkyl, e) phenyl, f) phenoxy, g) hydroxyl, h) C₁₋₅ hydroxyalkyl, i) C₁₋₅ haloalkyloxy, j) mercapto, k) C₁₋₅ alkylthio, 1) C₁₋₅ haloalkylthio, m) halogen, n) cyano, o) nitro, p) amino, q) C₁₋₅ alkylamino, r) C₂₋₁₀ dialkylamino, s) acyl, t) carboxyl, u) C₂₋₆ alkyloxycarbonyl, v) mesyl, w) trifluoromethanesulfonyl, and x) tosyl;
      R³ is hydrogen, halogen, C₁₋₅ alkyl, or C₁₋₅ alkoxy; R⁴ is -X- (CH₂)n -COOR⁵, and X is -O-, -S-, or -CH₂-; R⁵ is hydrogen or C₁₋₅ alkyl; and n is an integer that is 1, 2, or 3.
(B) The benzomorpholine derivative or pharmaceutically acceptable salt thereof according to (A) above represented by general formula (II), 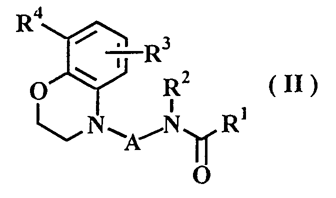 wherein A, R¹, R², R³, and R⁴ are as defined in the above formula (I).
(C) The benzomorpholine derivative or pharmaceutically acceptable salt thereof according to (A) or (B) above, wherein A is ethylene.
(D) The benzomorpholine derivative or pharmaceutically acceptable salt thereof according to any one of (A) to (C) above, wherein R¹ is unsubstituted aryl or heteroaryl, or aryl or heteroaryl substituted with one or a plurality of substituents which are as defined in (A) above.
(E) The benzomorpholine derivative or pharmaceutically acceptable salt thereof according to (D) above, wherein R¹ is unsubstituted phenyl, furyl, thienyl, or pyridyl, or phenyl, furyl, thienyl, or pyridyl substituted with one or a plurality of substituents which are as defined in (A) above.
(F) The benzomorpholine derivative or pharmaceutically acceptable salt thereof according to (E) above, wherein R¹ is unsubstituted phenyl, furyl, thienyl, or pyridyl, or phenyl, furyl, thienyl, or pyridyl substituted with one or a plurality of substituents independently selected from the following group,
   a) C₁₋₅ alkyl, b) C₁₋₅ alkoxy, c) C₁₋₅ haloalkyl, d) hydroxyl, e) C₁₋₅ haloalkyloxy, f) C₁₋₅ alkylthio, g) C₁₋₅ haloalkylthio, h) halogen, i) cyano, j) C₂₋₁₀ dialkylamino, k) acetyl, 1) C₂₋₆ alkyloxycarbonyl, m) mesyl, n) trifluoromethanesulfonyl, and o) tosyl.
(G) The benzomorpholine derivative or pharmaceutically acceptable salt thereof according to (F) above, wherein R¹ is unsubstituted phenyl, furyl, thienyl, or pyridyl, or phenyl, furyl, thienyl, or pyridyl substituted with one or a plurality of substituents independently selected from the following group,
   a) C₁₋₅ alkyl, b) C₁₋₅ alkoxy, c) C₁₋₅ haloalkyl, d) hydroxyl, h) halogen, and i) cyano.
(H) The benzomorpholine derivative or pharmaceutically acceptable salt thereof according to (A) to (G) above, wherein R² is unsubstituted phenyl or pyridyl, or phenyl or pyridyl substituted with one or a plurality of substituents which are as defined in (A) above.
(I) The benzomorpholine derivative or pharmaceutically acceptable salt thereof according to (H) above, wherein R² is unsubstituted phenyl or pyridyl, or phenyl or pyridyl substituted with one or a plurality of substituents independently selected from the following group,
   a) C₁₋₅ alkyl, b) C₁₋₅ alkoxy, c) C₁₋₅ haloalkyl, d) hydroxyl, e) C₁₋₅ haloalkyloxy, f) C₁₋₅ alkylthio, g) C₁₋₅ haloalkylthio, h) halogen, i) cyano, j) amino, k) C₂₋₁₀ dialkylamino, 1) acyl, m) C₂₋₆ alkyloxycarbonyl, n) mesyl, o) trifluoromethanesulfonyl, and p) tosyl.
(J) The benzomorpholine derivative or pharmaceutically acceptable salt thereof according to (I) above, wherein R² is unsubstituted phenyl or pyridyl, or phenyl or pyridyl substituted with one or a plurality of substituents independently selected from the following group,
   a) C₁₋₅ alkyl, b) C₁₋₅ alkoxy, c) C₁₋₅ haloalkyl, d) C₁₋₅ haloalkyloxy, e) C₁₋₅ alkylthio, f) halogen, and g) C₂₋₁₀ dialkylamino.
(K) The benzomorpholine derivative or pharmaceutically acceptable salt thereof according to any one of (A) to (J) above, wherein X is -O-.
(L) A drug comprising the benzomorpholine derivative according to any one of (A) to (K) above as an active ingredient.
(M) A platelet aggregation inhibitor or prophylactic comprising the benzomorpholine derivative according to any one of (A) to (K) above as an active ingredient.
(N) The platelet aggregation inhibitor or prophylactic according to (M) above, which is used for treating or preventing thrombosis or diseases accompanying thrombus.
(O) The platelet aggregation inhibitor or prophylactic according to (N) above, wherein the thrombosis is thrombosis in coronary arteries, cerebral arteries, peripheral arteries, or peripheral veins.
(P) The platelet aggregation inhibitor or prophylactic according to (N) above, wherein the disease accompanying thrombus is myocardial infarction, unstable angina, cerebral infarction, transient ischemic attack, or chronic arterial occlusion.

The following terms used in this specification are as defined below unless otherwise specified.

"Benzomorpholine" means, unless otherwise specified, a hydrogenated and condensed heterocycle resulting from condensation of the morpholine ring with the benzene ring at position 2 and position 3 of the morpholine ring.

"Alkylene" means, unless otherwise specified, a divalent linear or branched saturated hydrocarbon group comprising carbon and hydrogen atoms. Examples of such a group include, but are not limited to, ethylene, trimethylene, propylene, tetramethylene, and ethyl ethylene.

"Alkenylene" means, unless otherwise specified, a divalent linear or branched unsaturated hydrocarbon group comprising carbon and hydrogen atoms and having at least one double bond. Alkenylene groups include cis or trans ((E) or (Z)) isomers generated by asymmetric carbons. Examples of such an alkenylene group include, but are not limited to, ethenylene, 1-propenylene, 2-propenylene, and 2-butenylene.

"Alkynylene" means, unless otherwise specified, a divalent linear or branched unsaturated hydrocarbon group comprising carbon and hydrogen atoms and having at least one triple bond. Examples of such an alkynylene group include, but are not limited to, ethynylene, 1-propynylene, and 2-propynylene.

"Aryl" means a monovalent aromatic hydrocarbon group having one or more rings wherein at least one ring is aromatic. Examples of such an aryl group include, but are not limited to, phenyl, naphthyl, biphenylyl, indanyl, anthryl, and phenanthryl.

"Heteroaryl" means a monovalent aromatic group having one or more rings, wherein one, two, or three heteroatoms (selected from nitrogen, oxygen, and sulfur) are incorporated within the rings. Examples of such a heteroaryl group include, but are not limited to, imidazolyl, oxazolyl, pyrazinyl, thienyl, furyl, pyridyl, quinolyl, benzofuryl, indolyl, pyrrolyl, and pyranyl.

"Alkyl" means, unless otherwise specified, a monovalent linear or branched saturated hydrocarbon group comprising carbon and hydrogen atoms. Examples of such a group include, but are not limited to, methyl, ethyl, propyl, isopropyl, butyl, isobutyl, sec-butyl, tert-butyl, and pentyl.

"Alkenyl" means, unless otherwise specified, a monovalent linear or branched unsaturated hydrocarbon group comprising carbon and hydrogen atoms and having at least one double bond. Alkenyl groups include cis or trans ((E) or (Z)) isomers generated by asymmetric carbons. Examples of such an alkenyl group include, but are not limited to, ethenyl, 1-propenyl, allyl, 1-butenyl, 2-butenyl, 2-pentenyl, and 1,3-butanedienyl.

"Alkynyl" means, unless otherwise specified, a monovalent linear or branched unsaturated hydrocarbon group comprising carbon and hydrogen atoms and having at least one triple bond. Examples of such an alkynyl group include, but are not limited to, ethynyl, 1-propynyl, propargyl (2-propynyl), and 3-butynyl.

"Alkoxy" means an -OR group, wherein R is alkyl as defined here. Examples of such an alkoxy group include, but are not limited to, methoxy, ethoxy, propoxy, isopropoxy, butoxy, and t-butoxy.

"Cycloalkyl" means, unless otherwise specified, a monovalent saturated hydrocarbon ring group comprising carbon and hydrogen atoms and having at least one or more rings. Examples of such a cycloalkyl group include cyclopropyl, cyclobutyl, cyclopentyl, cyclohexyl, and cycloheptyl.

"Cycloalkenyl" means, unless otherwise specified, a monovalent unsaturated hydrocarbon ring group comprising carbon and hydrogen atoms, having at least one double bond, and containing one or more rings. Examples of such a cycloalkenyl group include cyclopentenyl, cyclohexenyl, and cycloheptenyl.

"Haloalkyl" means an alkyl group as defined here, which is substituted with one or more halogen atoms as defined here at an arbitrary position(s). Examples of such an haloalkyl group include, but are not limited to, trifluoromethyl, 2,2,2-trifluoroethyl, and 2,2,2-trichloroethyl.

"Hydroxyalkyl" means an alkyl group as defined here, which is substituted with one or more hydroxyl groups at an arbitrary position(s). Examples of such a hydroxyalkyl group include, but are not limited to, hydroxymethyl, 2-hydroxyethyl, 2-hydroxypropyl, and 3-hydroxypropyl.

"Haloalkyloxy" means an alkoxy group as defined here, which is substituted with one or more halogen atoms as defined here at an arbitrary position(s). Examples of such a haloalkyloxy group include, but are not limited to, fluoromethoxy, difluoromethoxy, trifluoromethoxy, and 2,2,2-trifluoroethoxy.

"Alkylthio" means a -SR group, wherein R is alkyl as defined here. Examples of such an alkylthio group include, but are not limited to, methylthio and ethylthio.

"Haloalkylthio" means an alkylthio group as defined here, which is substituted with one or more halogen atoms as defined here at an arbitrary position(s). Examples of such a haloalkylthio include, but are not limited to, fluoromethylthio, difluoromethylthio, trifluoromethylthio, and 2,2,2-trifluoroethylthio.

"Halogen" means fluoro, chloro, bromo, or iodo.

"Alkylamino" means an amino group, which is substituted with one alkyl group as defined here. Examples of such an alkylamino group include, but are not limited to, methylamino, ethylamino, propylamino, and isopropylamino.

"Dialkylamino" means an amino group, which is substituted with two independent alkyl groups as defined here. Examples of such a dialkylamino group include, but are not limited to, dimethylamino, diethylamino, and ethylmethylamino.

"Acyl" means a C₁₋₆ aliphatic acyl group and an aromatic acyl group. Examples of such a C₁₋₆ aliphatic acyl group include formyl, acetyl, propionyl, butyryl, isobutyryl, isobutyryl, valeryl, isovaleryl, and pivaloyl. Examples of such an aromatic acyl group include benzoyl and naphthoyl.

"Alkyloxycarbonyl" means a -COOR group, wherein R is alkyl as defined here. Examples of such an alkyloxycarbonyl group include, but are not limited to, methoxycarbonyl, ethoxycarbonyl, isopropoxycarbonyl, butoxycarbonyl, and t-butoxycarbonyl.

"Pharmaceutically acceptable" means that an object is generally safe, is non-toxic, and can be useful in preparation of a pharmaceutical preparation that is acceptable as an animal drug or a human drug having no problems biologically and in terms of other points.

"Pharmaceutically acceptable salt" means a salt that is pharmaceutically acceptable and has desired pharmacological activity of a parent compound. Examples of such a salt are as follows:
acid addition salts generated with inorganic acids such as hydrochloric acid, sulfuric acid, nitric acid, and phosphoric acid, or acid addition salts generated with organic acids such as acetic acid, propionic acid, glycolic acid, butyric acid, malonic acid, succinic acid, maleic acid, fumaric acid, tartaric acid, citric acid, benzoic acid, cinnamic acid, mandelic acid, methanesulfonic acid, lauryl sulfate, glutamic acid, hydroxynaphthoic acid, salicylic acid, stearic acid, and muconic acid;
base addition salts generated by substitution of acidic protons existing in parent compounds with alkaline metal ions, alkaline earth metal ions, ammonium cations, or the like (where salts of other metals such as cationic alminium, zinc, or iron are absolutely included in the present invention); or
base addition salts generated by coordinate bonding of acidic protons existing in parent compounds with organic bases.

Examples of inorganic bases to be used for generation of the base addition salts include lithium hydroxide, sodium hydroxide, potassium hydroxide, calcium hydroxide, aluminium hydroxide, and sodium carbonate.

An organic base to be used for generation of the base addition salt is primary, secondary, or tertiary amine. Examples of appropriate amine include:
methylamine, dimethylamine, triethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, and diethylenetriamine;
aliphatic, alicyclic, and heterocyclic amines that are primary, secondary, or tertiary amines containing a maximum of 18 carbon atoms, such as 1-methylpiperidine, 4-ethylmorpholine, 1-isopropylpyrrolidine, 2-methylpyrrolidine, 1,4-dimethylpiperazine, and 2-methylpiperidine;
water-soluble amines or amines containing hydrophilic groups, such as mono-, di-, or triethanolamine, ethyldiethylamine, N-butylethanolamine, 2-amino-1-butanol, 2-amino-2-ethyl-1, 3-propanediol, tris(hydroxymethyl)aminomethane, N-phenylethanolamine, N-phenyldiethanolamine, galactosamine, meglumine (N-methylglucamine), N-methylglucosamine, ephedrine, phenylephrine, epinephrine, and procaine; and
basic amino acids, such as lysine and arginine.

In the benzomorpholine derivative represented by general formula (I) or (II) of the present invention, regarding A, examples of C₂₋₄ alkylene include ethylene, trimethylene, propylene, tetramethylene, and ethylethylene. Examples of C₂₋₄ alkenylene include ethenylene, 1-propenylene, 2-propenylene, and 2-butenylene. Examples of C₂₋₄ alkynylene include ethynylene, 1-propynylene, and 2-propynylene. Of these, ethylene, trimethylene, and tetramethylene are preferable. Ethylene and trimethylene are further preferable. Ethylene is particularly preferable.

Regarding R¹, examples of aryl include phenyl, naphthyl, biphenylyl, and indanyl. Examples of heteroaryl include imidazolyl, oxazolyl, pyrazinyl, thienyl, furyl, pyridyl, quinolyl, benzofuryl, indolyl, pyrrolyl, and pyranyl. Examples of C₁₋₅ alkyl include methyl, ethyl, propyl, isopropyl, butyl, isobutyl, sec-butyl, tert-butyl, and pentyl. Examples of C₃₋₈ cycloalkyl include cyclopropyl, cyclobutyl, cyclopentyl, cyclohexyl, and cycloheptyl. Examples of C₂₋₁₀ alkenyl include ethenyl, 1,3-butanedienyl, and allyl. Examples of C₄₋₁₀ cycloalkenyl include 1-cyclopentenyl, 1-cyclohexenyl, and cyclohexylidenemethyl. Examples of C₂₋₁₀ alkynyl include ethynyl, propargyl, and 3-butynyl. Of these, phenyl, furyl, thienyl, pyridyl, cyclopropyl, ethenyl, ethynyl, 1-cyclopentenyl, 1-cyclohexenyl, and cyclohexylidenemethyl are preferable. Phenyl, furyl, thienyl, pyridyl, ethenyl, 1-cyclopentenyl, 1-cyclohexenyl, and cyclohexylidenemethyl are further preferable. Phenyl, furyl, thienyl, pyridyl, ethenyl, and 1-cyclohexenyl are particularly preferable.

Regarding substituents when R¹ is aryl or heteroaryl, examples of C₁₋₅ alkyl include methyl and isopropyl. Examples of C₁₋₅ alkoxy include methoxy, ethoxy, and isopropoxy. Examples of C₃₋₈ cycloalkyl include cyclopropyl, cyclopentyl, cyclohexyl, and cycloheptyl. Examples of C₁₋₅ haloalkyl include trifluoromethyl and 2,2,2-trifluoroethyl. Examples of C₁₋₅ hydroxyalkyl include hydroxymethyl and 2-hydroxyethyl. Examples of C₁₋₅ haloalkyloxy include trifluoromethoxy and 2,2,2-trifluoroethoxy. Examples of C₁₋₅ alkylthio include methylthio and ethylthio. Examples of C₁₋₅ haloalkylthio include trifluoromethylthio and 2,2,2-trifluoroethylthio. Examples of halogen include fluoro, chloro, bromo, and iodo. Examples of C₁₋₅ alkylamino include methylamino, ethylamino, and isopropylamino. Examples of C₂₋₁₀ dialkylamino include dimethylamino, diethylamino, and ethylmethylamino. Among acyl, examples of C₁₋₆ aliphatic acyl groups include formyl, acetyl, propionyl, butyryl, isobutyryl, isobutyryl, valeryl, isovaleryl, and pivaloyl. Examples of aromatic acyl groups include benzoyl and naphthoyl. Examples of C₂₋₆ alkyloxycarbonyl include methoxycarbonyl, ethoxycarbonyl, butoxycarbonyl, and t-butoxycarbonyl. Furthermore, other examples of substituents include phenyl, phenoxy, hydroxyl, mercapto, cyano, nitro, amino, carboxyl, mesyl (methanesulfonyl), trifluoromethanesulfonyl, and tosyl. Of these, same or different one to three methyl, methoxy, trifluoromethyl, hydroxyl, trifluoromethoxy, methylthio, trifluoromethylthio, fluoro, chloro, bromo, cyano, dimethylamino, acetyl, methoxycarbonyl, mesyl, and trifluoromethanesulfonyl substituents are preferable. Same or different one to three methyl, methoxy, trifluoromethyl, hydroxyl, fluoro, chloro, bromo, and cyano substituents are more preferable. Same or different one to three trifluoromethyl, fluoro, and chloro substituents are particularly preferable.

Regarding substituents when R¹ is C₁₋₅ alkyl, C₃₋₈ cycloalkyl, C₂₋₁₀ alkenyl, C₄₋₁₀ cycloalkenyl, or C₂₋₁₀ alkynyl, examples of C₁₋₅ alkyl include methyl and ethyl. Examples of C₃₋₈ cycloalkyl include cyclopropyl, cyclobutyl, cyclopentyl, and cyclohexyl. Examples of C₁₋₅ haloalkyl include trifluoromethyl and 2,2,2-trifluoroethyl. Examples of halogen include fluoro, chloro, and bromo. Furthermore, other examples of substituents include phenyl and hydroxyl. Of these, same or different one to three phenyl, hydroxyl, methyl, cyclohexyl, trifluoromethyl, fluoro, and chloro substituents are preferable. Same or different one to three methyl, cyclohexyl, trifluoromethyl, fluoro, and chloro substituents are more preferable. Same or different one to three methyl, trifluoromethyl, fluoro, and chloro substituents are particularly preferable.

Regarding R², examples of aryl include phenyl, naphthyl, biphenylyl, and indanyl. Examples of heteroaryl include imidazolyl, oxazolyl, pyrazinyl, thienyl, furyl, pyridyl, quinolyl, benzofuryl, indolyl, pyrrolyl, and pyranyl. Of these, phenyl, naphthyl, imidazolyl, thienyl, furyl, pyridyl, and indolyl are preferable. Phenyl, thienyl, furyl, and pyridyl are more preferable. Phenyl and pyridyl are particularly preferable. Substituents when R² is aryl or heteroaryl are similar to the case where R¹ is aryl or heteroaryl. Of these, same or different one to three methyl, methoxy, trifluoromethyl, hydroxyl, trifluoromethoxy, methylthio, trifluoromethylthio, fluoro, chloro, bromo, iodo, cyano, amino, dimethylamino, acetyl, methoxycarbonyl, mesyl, and trifluoromethanesulfonyl substituents are preferable. Same or different one to three methyl, methoxy, trifluoromethyl, trifluoromethoxy, methylthio, fluoro, chloro, bromo, iodo, and dimethylamino substituents are more preferable. Same or different one to three methyl, methoxy, trifluoromethyl, trifluoromethoxy, methylthio, fluoro, chloro, and bromo substituents are particularly preferable.

Examples of R³ include hydrogen and halogen such as fluoro, chloro, bromo, and iodo. Examples of C₁₋₅ alkyl include methyl, ethyl, and isopropyl. Examples of C₁₋₅ alkoxy include methoxy, ethoxy, and isopropoxy. Of these, hydrogen and same or different one to three fluoro, chloro, bromo, methyl, isopropyl, methoxy, and isopropoxy substituents are preferable. Hydrogen and same or different one to three fluoro, chloro, methyl, and methoxy substituents are more preferable. Same or different one to three fluoro and methyl substituents are particularly preferable.

Regarding R⁴, -O- and -CH₂- are preferable as X, and -O- is particularly preferable. Examples of R⁵ include hydrogen and C₁₋₅ alkyl such as methyl, ethyl, propyl, butyl, and tert-butyl. Of these, hydrogen, methyl, ethyl, and butyl are preferable. Hydrogen and methyl are more preferable. Hydrogen is particularly preferable. As n, 1 or 2 is preferable, and 1 is particularly preferable.

Examples of pharmaceutically acceptable salts include sodium salt, potassium salt, calcium salt, methylamine salt, dimethylamine salt, triethylamine salt, α-phenylethylamine salt, β-phenylethylamine salt, ethylenediamine salt, monoethanolamine salt, diethanolamine salt, triethanolamine salt, and meglumine salt. Sodium salt, potassium salt, calcium salt, methylamine salt, dimethylamine salt, triethylamine salt, α-phenylethylamine salt, β-phenylethylamine salt, diethanolamine salt, and meglumine salt are preferable. Sodium salt, potassium salt, diethanolamine salt, and meglumine salt are particularly preferable.

Among the benzomorpholine derivatives of the present invention, various optical isomers are present when the derivative has an asymmetric carbon within the molecule. Furthermore, various diastereomers are present when the derivative has at least two asymmetric carbons. The present invention also encompasses these optical isomers and diastereomers. Furthermore, the present invention also encompasses cis-trans isomers.

Specific examples of the benzomorpholine derivatives represented by general formula (I) or (II) of the present invention are shown in Tables 1 to 7, but the present invention is not limited by these examples.

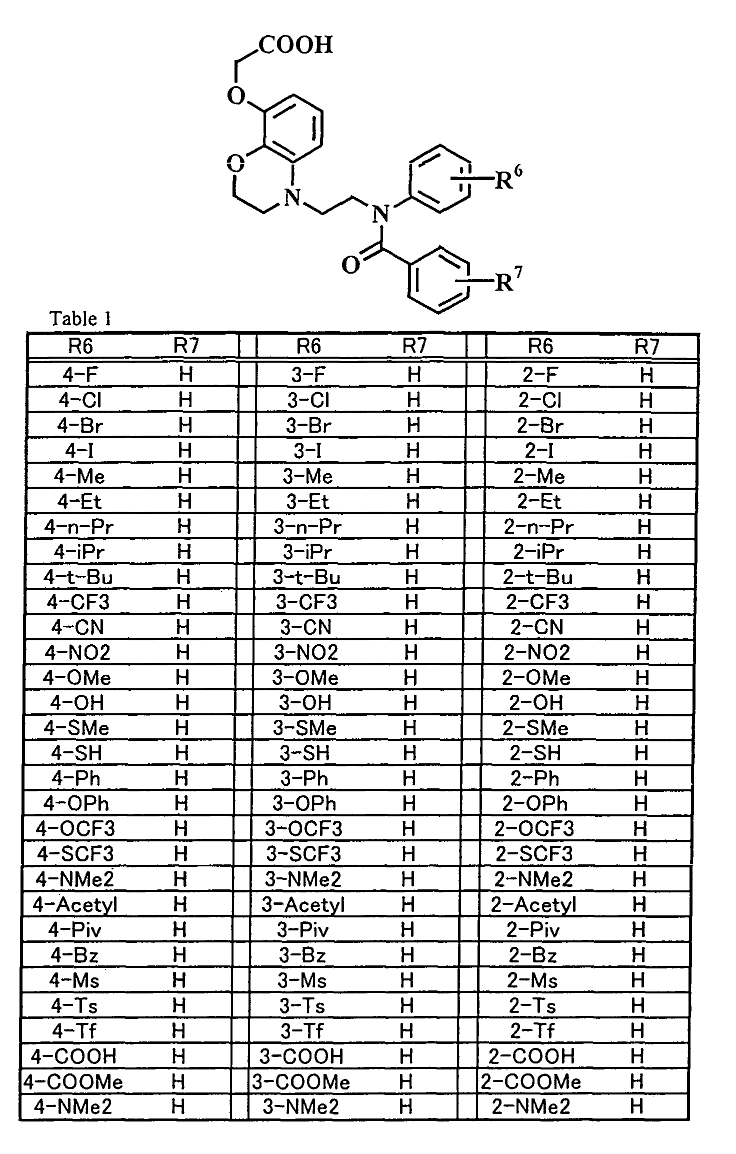

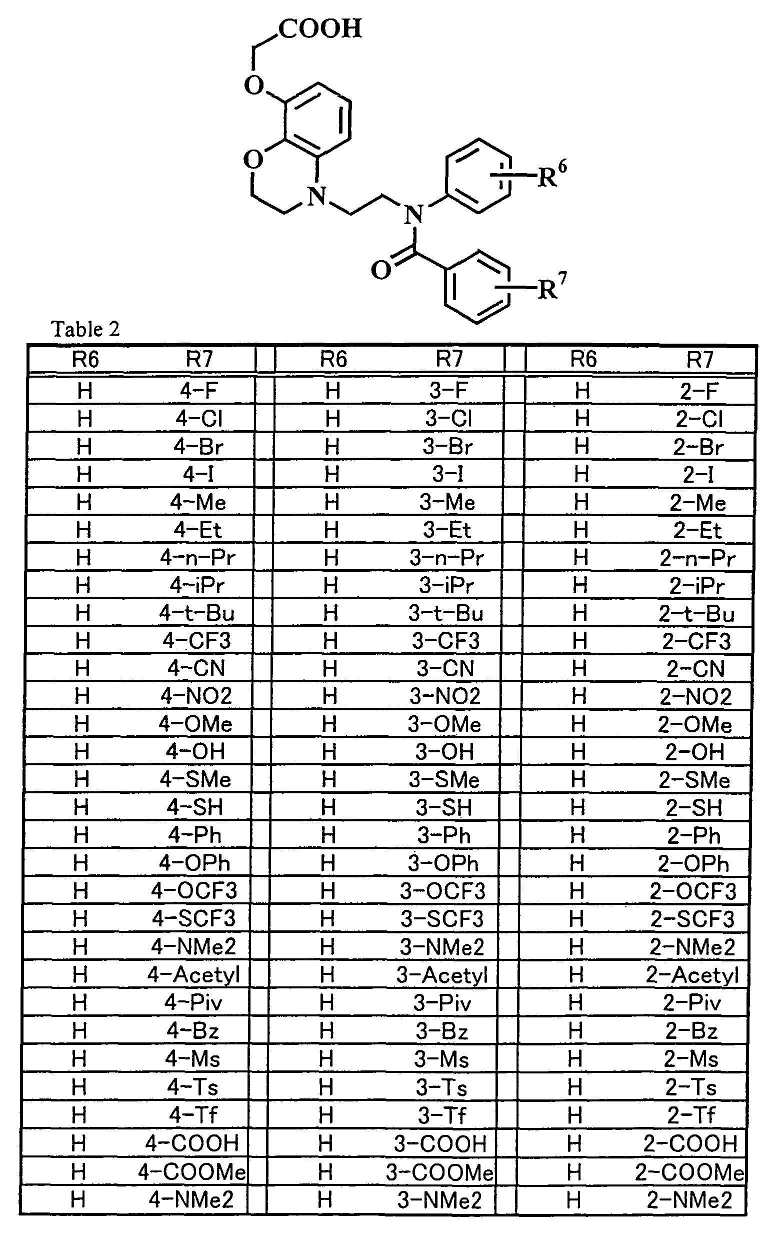

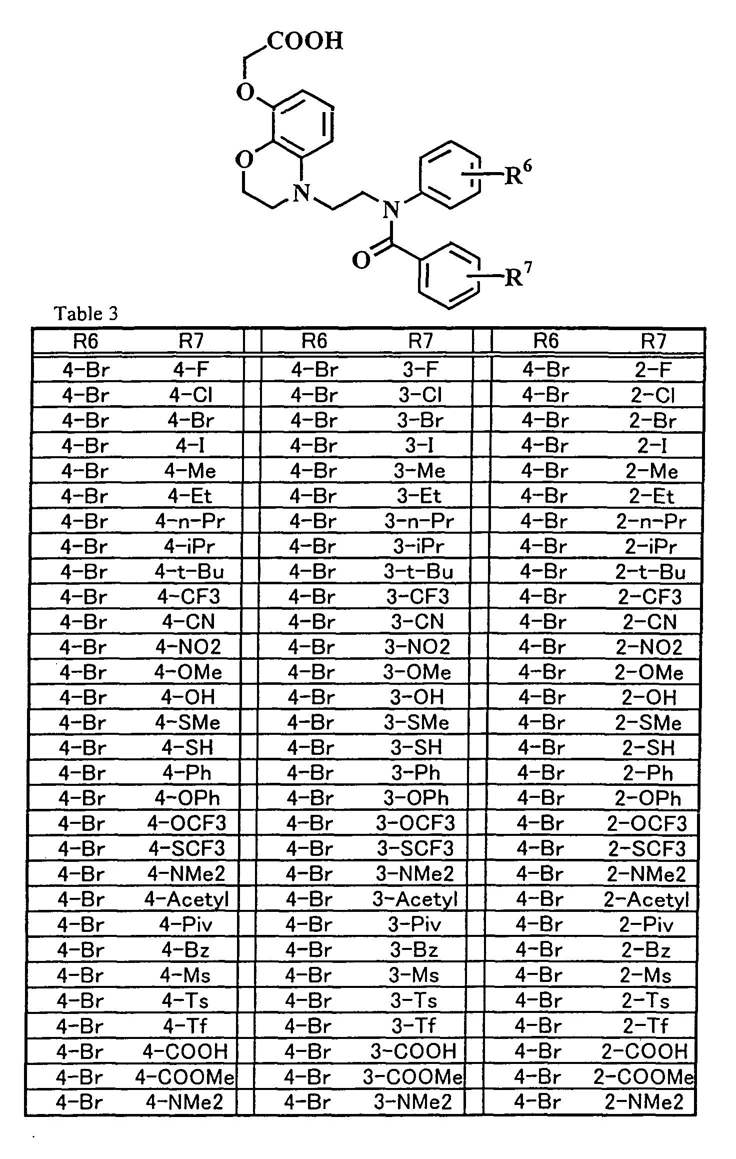

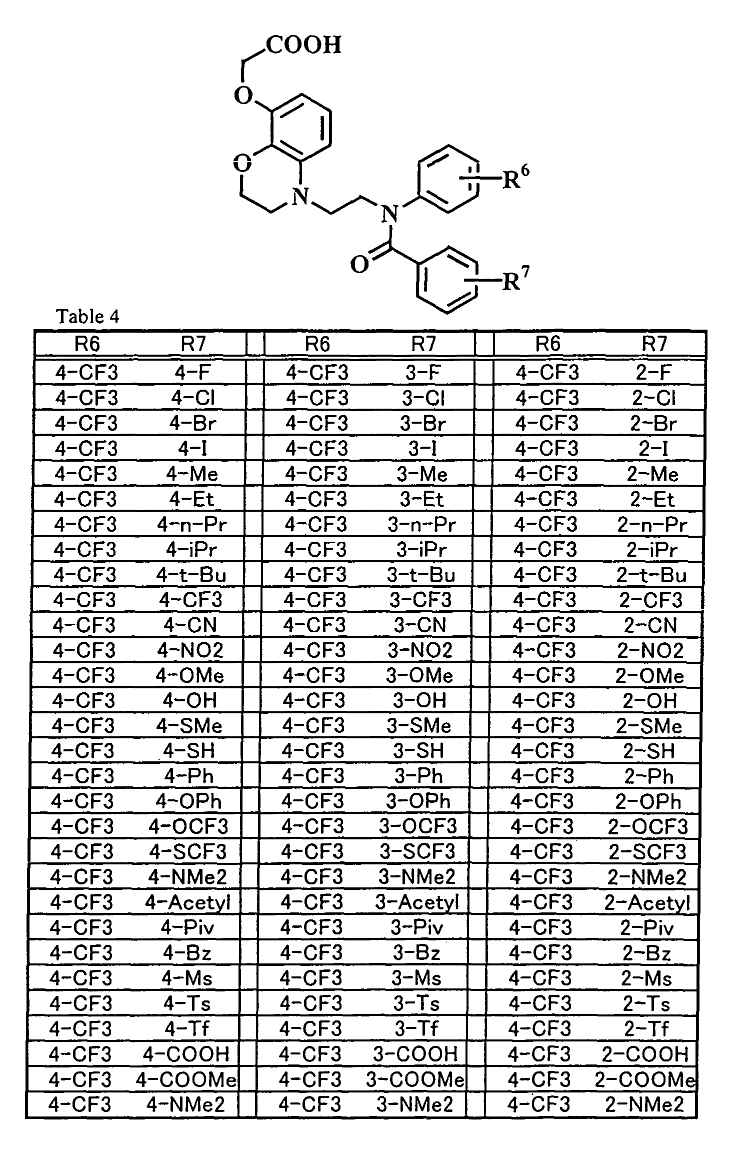

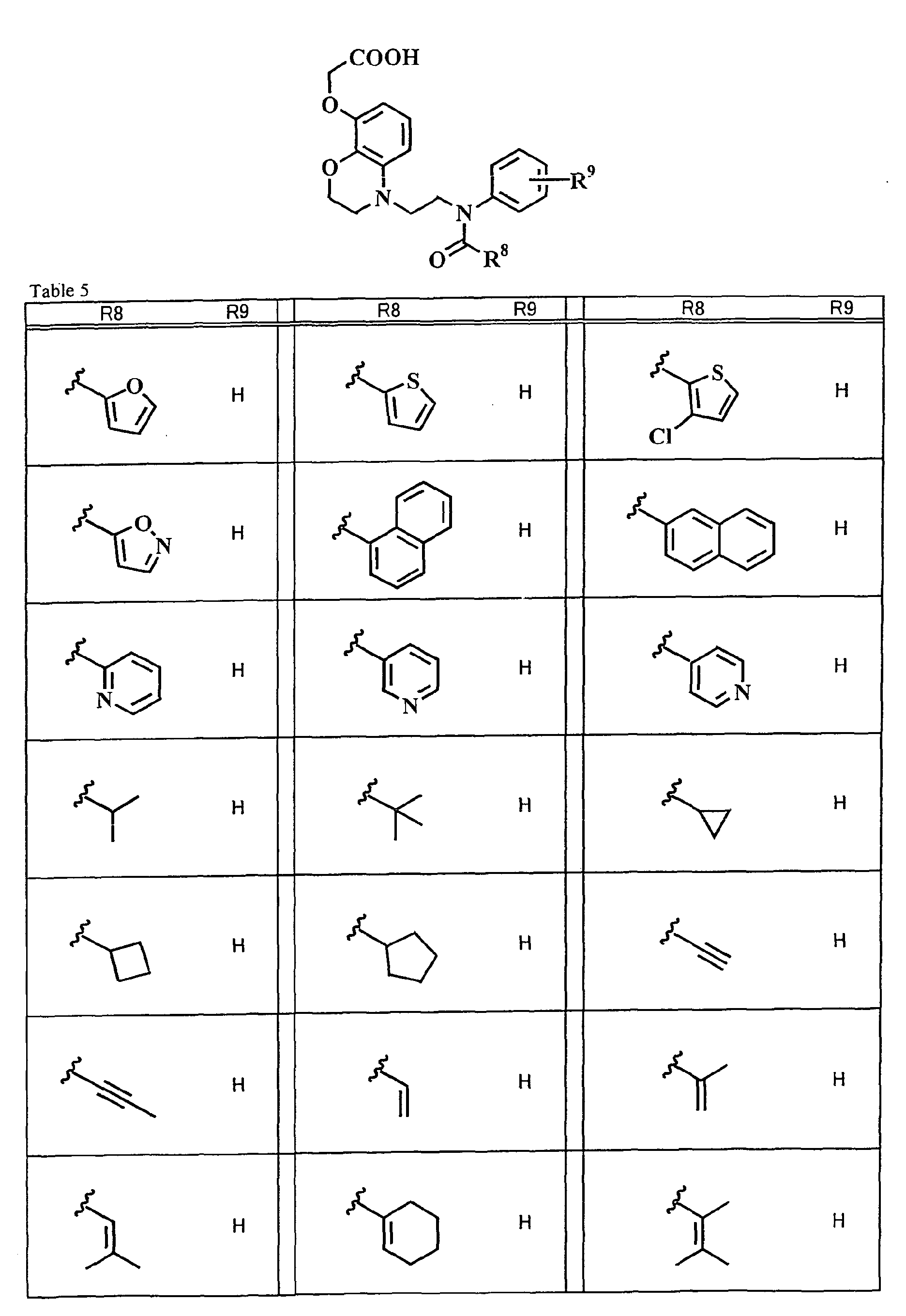

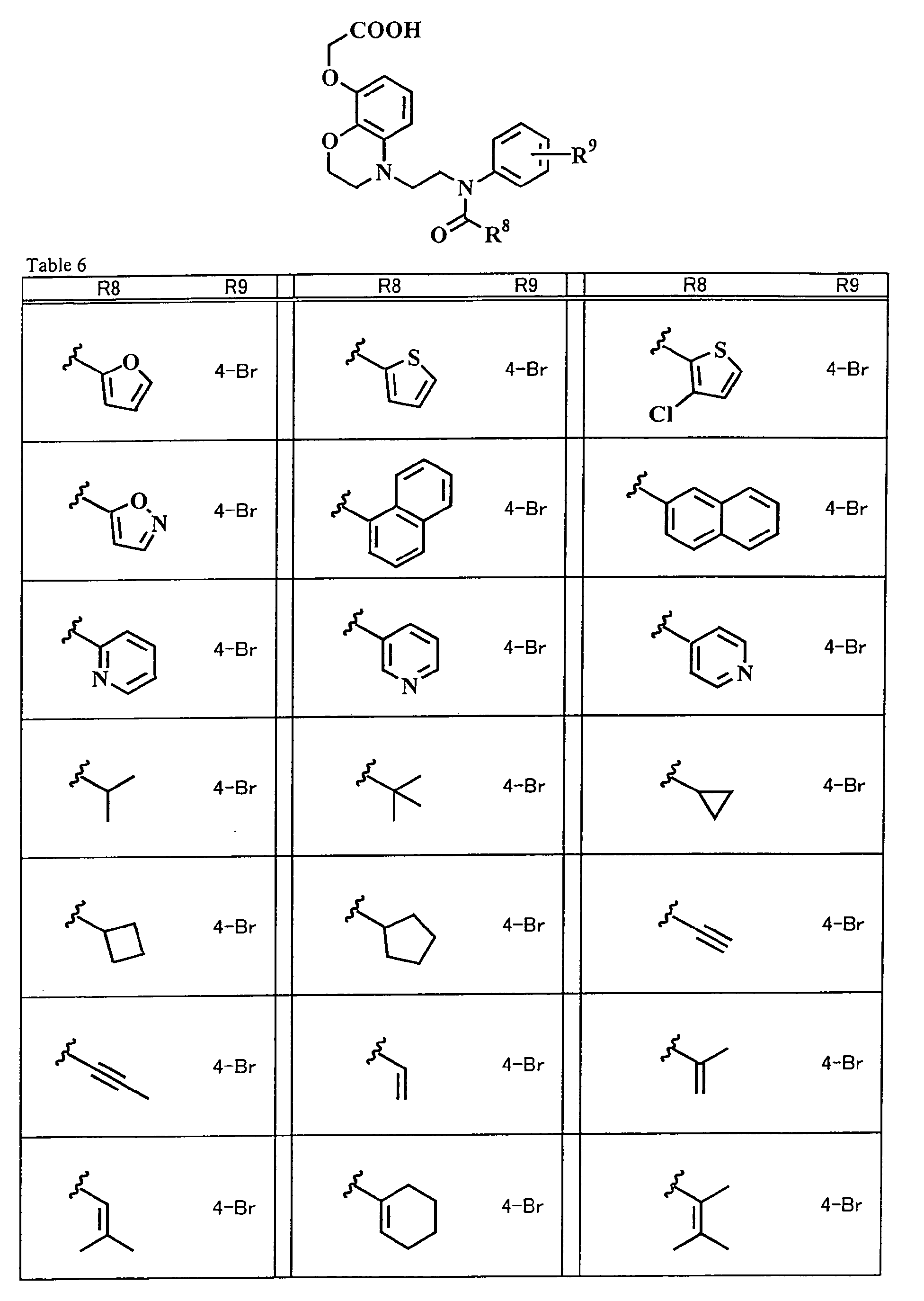

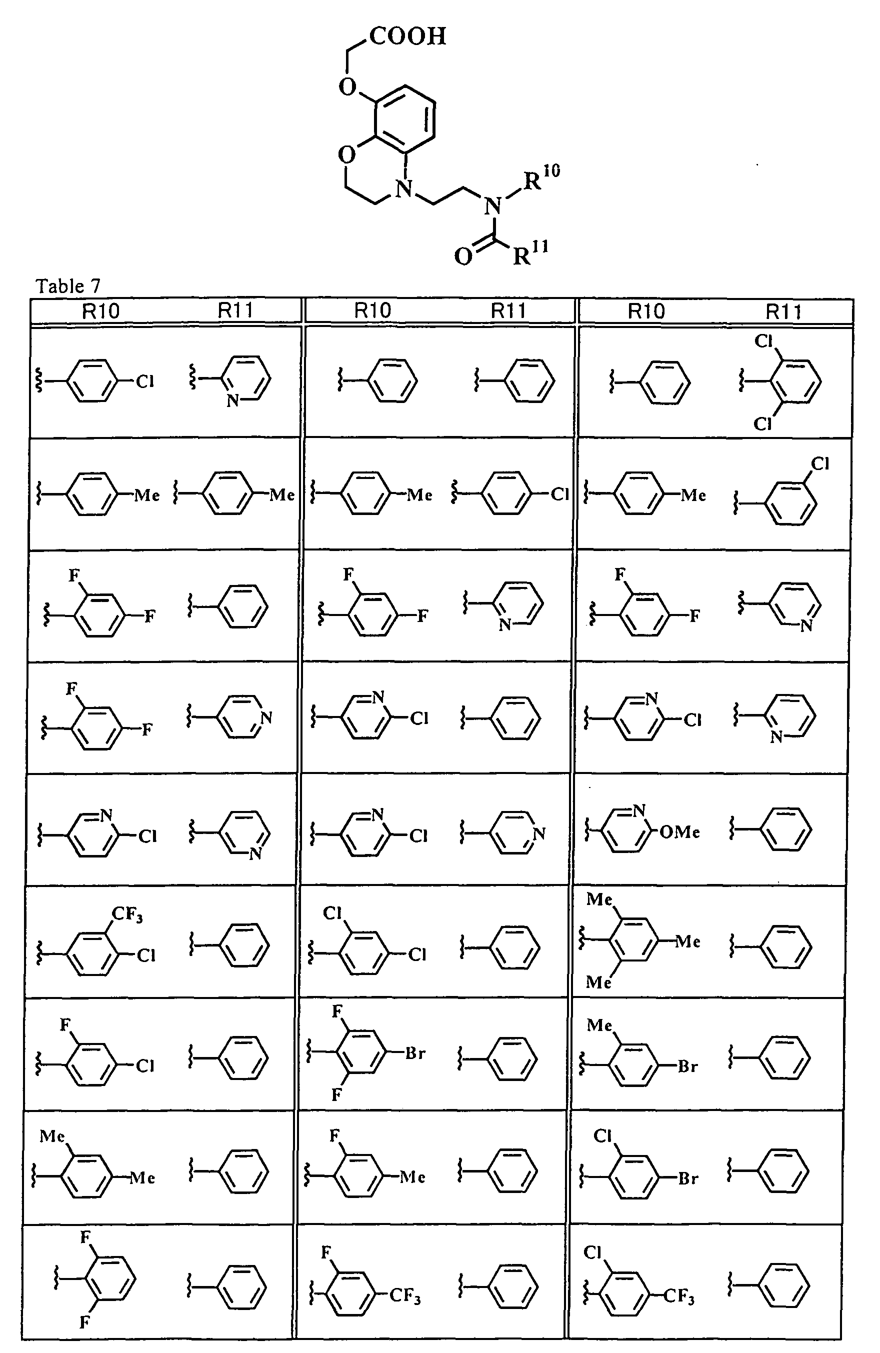

The meanings of abbreviations in Tables 1 to 7 are as follows:
Piv: pivaloyl, Bz: benzoyl, Ms: mesyl (SO₂Me) Ts: tosyl, and Tf: trifluoromethanesulfonyl (the same meanings are applied for the following abbreviations).

The benzomorpholine derivatives of the present invention can be produced by methods shown in the following synthetic schemes. Starting substances and reagents to be used for production of these compounds can be commercially available or can be synthesized by methods known by persons skilled in the art according to procedures described in references such as Organic Reaction (Wiley & Sons), Fieser and Fieser's Reagent for Organic Synthesis (Wiley & Sons), and the like. The following schemes merely illustrate some methods with which the benzomorpholine derivatives of the present invention can be produced. The scope of the present invention is not limited by these methods.

Unless otherwise specified, reactions described here are carried out under atmospheric pressure at a temperature between -100°C and 150°C, more preferably between -20°C and 125°C, and particularly preferably between 0°C and 100°C.

Of the benzomorpholine derivatives of the present invention represented by general formula (I), for example, a benzomorpholine derivative represented by general formula (Ia), 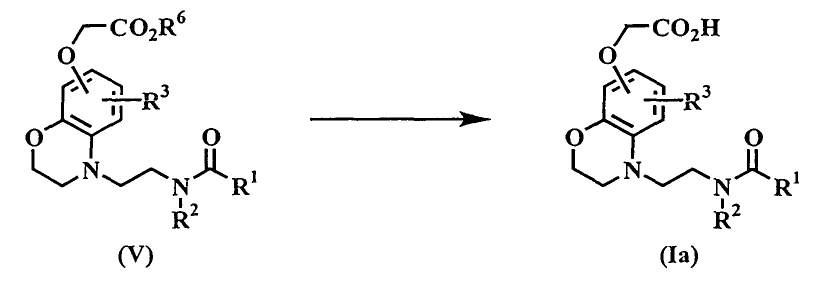 wherein, the symbols represents the same as described above,
that is the benzomorpholine derivative represented by general formula (I), wherein A is ethylene, X is O, R⁵ is hydrogen, and n is 1, can be produced by hydrolysis of compounds represented by general formula (V) (wherein R⁶ represents C₁₋₅ alkyl and other symbols have the same meanings as those given above) under alkaline conditions. Ester hydrolysis under alkaline conditions is known and is carried out in an organic solvent that is miscible with water, such as tetrahydrofuran, dioxane, methanol, ethanol, dimethoxyethane, or a mixed solvent thereof, using aqueous alkaline such as an aqueous sodium hydroxide or an aqueous potassium hydroxide at a temperature between -10°C and 70°C.

The benzomorpholine derivative represented by general formula (V) (symbols in the formula have the same meanings as those given above) can be produced by reacting an amide compound represented by general formula (III) (symbols in the formula have the same meanings as those given above) with a benzomorpholine derivative represented by general formula (IV) (wherein Y represents halogen, tosyloxy, or mesyloxy and other symbols have the same meanings as those given above).

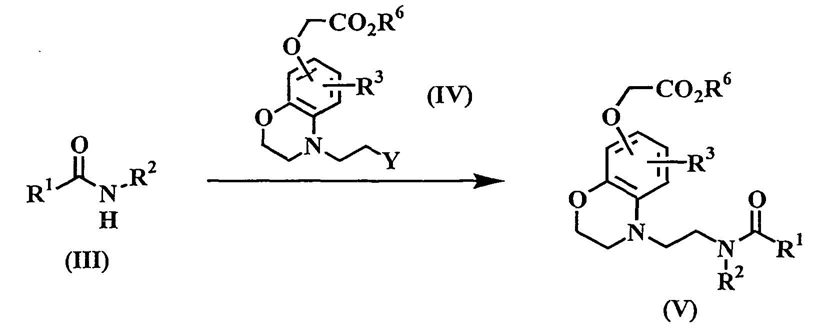

N-alkylation reaction of amide is known and is carried out in dimethylformamide, tetrahydrofuran, dioxane, acetone, dimethoxyethane, or a mixed solvent thereof, by treating the amide compound represented by general formula (III) with a base such as sodium hydride, potassium hydride, potassium t-butoxide, or lithium diisopropylamide (LDA) and then adding the benzomorpholine derivative represented by general formula (IV) generally at a temperature between -20°C and 70°C.

Furthermore, the benzomorpholine derivative represented by general formula (IV), for example, a benzomorpholine derivative (IVa) that is the benzomorpholine derivative represented by general formula (IV), wherein Y is mesyloxy, can be produced by mesylation of a benzomorpholine derivative represented by general formula (VII) (symbols in the formula have the same meanings as those given above) (wherein Ms in the benzomorpholine derivative (IVa) represents mesyl).

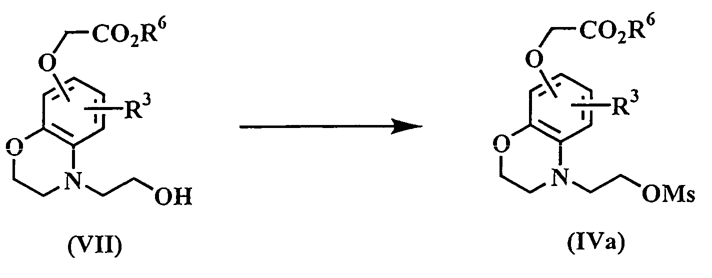

O-mesylation reaction is known and carried out in dimethylformamide, tetrahydrofuran, dioxane, acetone, dimethoxyethane, or a mixed solvent thereof, in the presence of a base such as triethylamine or morpholine, by treating the benzomorpholine derivative represented by general formula (VII) with mesyl chloride or methanesulfonic acid anhydride generally at a temperature between -20°C and 70 °C.

Furthermore, the benzomorpholine derivative represented by general formula (V) can also be produced by acylation of the benzomorpholine derivative represented by general formula (VI) (symbols in the formula have the same meanings as those given above).

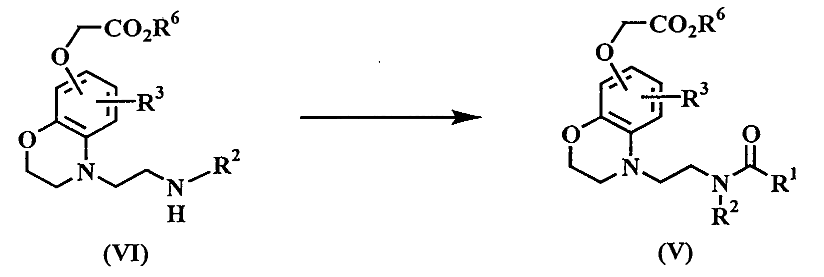

N-acylation reaction is known and carried out in dimethylformamide, tetrahydrofuran, dioxane, acetone, dimethoxyethane, or a mixed solvent thereof in the presence of a base such as triethylamine or morpholine by treating the benzomorpholine derivative represented by general formula (VI) with the corresponding acid chloride (R¹COCl) or acid anhydride ((R¹CO)₂O) generally at a temperature between -20°C and 70°C. Alternatively, the reaction is carried out in dimethylformamide, tetrahydrofuran, dioxane, dimethoxyethane, or a mixed solvent thereof, in the presence of an appropriate condensing agent such as dicyclohexylcarbodiimide (DCC) by treating the benzomorpholine derivative represented by general formula (VI) with the corresponding carboxylic acid (R¹COOH) generally at a temperature between -20°C and 70°C.

The benzomorpholine derivative represented by general formula (VI) can be produced by N-alkylation of primary amine (H₂N-R²) with the benzomorpholine derivative represented by general formula (IVa) (symbols in the formula have the same meanings as those given above).

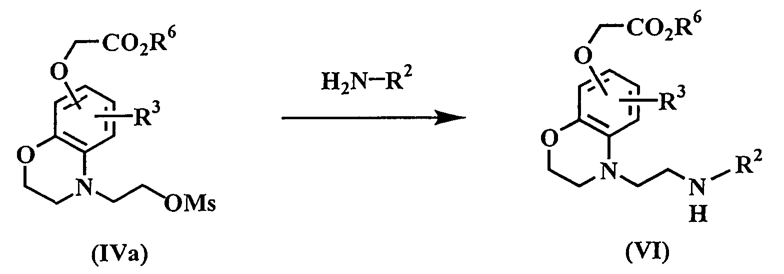

N-alkylation reaction is known and carried out in dimethylformamide, acetonitrile, toluene, or a mixed solvent thereof in the presence of a base such as triethylamine or diisopropylethylamine by treating the corresponding primary amine (H₂N-R²) with the benzomorpholine derivative represented by general formula (IVa) generally at a temperature between -20°C and 150°C.

Compounds and reagents represented by general formulae (III), (IV), and (VII) can be produced by methods which are known by themselves or are self-evident to persons skilled in the art. Of these, the production method of the benzomorpholine derivative (VII) is described in WO 00/07992. In each reaction in this specification, reaction products can be purified by general purification means, such as distillation under normal pressure or reduced pressure; high performance liquid chromatography, thin-layer chromatography or column chromatography which uses silica gel or magnesium silicate; or by a method such as washing or recrystallization. Purification may be carried out for every reaction or after completion of several reactions.

The benzomorpholine derivatives of the present invention can be used as drugs. For example, because their strong inhibitory effect of platelet aggregation, the benzomorpholine derivatives of the present invention or compositions comprising the benzomorpholine derivatives of the present invention are effective for preventing and treating various diseases resulting from thrombus in mammals, particularly humans.

More specifically, the benzomorpholine derivatives of the present invention are effective as prophylactics and therapeutic agents for thrombosis, particularly for thrombosis in coronary arteries, cerebral arteries, or peripheral arteries, or diseases accompanying thrombus.

Examples of thrombosis described herein include arterial thrombosis such as coronary thrombosis and pulmonary thrombosis, venous thrombosis such as deep vein thrombosis, thrombosis in the heart such as mural thrombosis, and thrombosis in extracorporeal circulation path. Examples of diseases accompanying thrombus include myocardial infarction, unstable angina, cerebral infarction, transient ischemic attack, acute or chronic arterial occlusion, restenosis after PTCA, disseminated intravascular coagulation (DIC), cerebral embolism, and pulmonary embolism.

The present invention encompasses pharmaceutical composition comprising pharmaceutically acceptable salts of benzomorpholine derivatives, one or more types of pharmaceutically acceptable carriers, and, depending on the case, other ingredients for treatment and/or prevention.

In general, the benzomorpholine derivatives of the present invention are administered in therapeutically effective amounts by any acceptable administration method for medicaments useful for similar applications. The benzomorpholine derivatives of the present invention are generally administered by intravenous injection, intraarterial injection, intramuscular injection, subcutaneous injection, transdermal administration, transpulmonary administration, transnasal administration, instillation, rectal administration, or oral administration.

In the case of general transdermal administration, transpulmonary administration, transnasal administration, rhinenchysis, rectal administration, or oral administration, the derivative is administered 1 to 4 separate times a day within the range between 0.1 µg/kg/day and 100 mg/kg/day. In the case of intravenous or intraarterial drip infusion, preferable results can be obtained by administering the derivative in an amount within the range between 1 ng/kg/minute and 1 mg/kg/minute. In the case of general intravenous injection, intraarterial injection, intramuscular injection, and subcutaneous injection, the derivative is administered 1 to 4 separate times a day within the range between 0.01 µg/kg/day and 100 mg/kg/day. In the case of such administration, the dose is selected from the above range in consideration of age, gender, and the conditions of each patient, the number of instances of administration of a medicament, and the like.

If necessary, pharmaceutically acceptable additives may be added to the benzomorpholine derivatives of the present invention. The derivatives can be orally administered in the form of solids containing, in addition to excipients such as starch, lactose, sucrose, D-mannitol, sorbitol, or microcrystalline cellulose, binders, disintegrating agents, coating agents, stabilizers, preservatives, solubilizing agents, colorants, lubricants, and the like. The benzomorpholine derivatives of the present invention may be administered parenterally in the form of sterile pharmaceutical preparations and may contain as additives, isotonizing agents such as sodium chloride, D-mannitol, xylitol, or glucose, pH regulators, and solubilizers. Since the benzomorpholine derivatives of the present invention have chemical structural stability, the forms of relevant pharmaceutical products are not specifically limited, as long as they are in pharmaceutically acceptable forms for administration. Wide-ranging formulations are selectable. Examples include pharmaceutical preparations for the above oral administration, such as tablets, powders, granules, capsules, soft capsules, and syrups, various injections, suppositories, ointments, gels, aerosols, suspensions, liquid preparations, tapes, and lotions.

The benzomorpholine derivatives of the present invention can also be used in combination with, for example, other anti-thrombotic drugs, or prophylactics or therapeutic agents for other diseases (e.g., hypertension, diabetes mellitus, hyperlipemia, and coronary vasodilator).

Examples of other anti-thrombotic drugs include ADP receptor antagonists such as ticlopidine, clopidogrel, and CS-747, phosphodiesterase inhibitors such as cilostazol, pentoxifylline, and dipyridamole, 5-HT receptor antagonists such as sarpogrelate, GpIIb/IIIa antagonists such as abciximab, tirofiban, and roxifiban, thromboxane synthase inhibitors such as ozagrel, Xa factor inhibitors such as fondaparinux, thrombin inhibitors such as argatroban, low molecular heparin such as enoxaparin and reviparin, thrombolytic agents such as t-PA, urokinase, and streptokinase. Furthermore, aspirin, heparin, and the like are also included.

Examples of prophylactics or therapeutic agents for hypertension include α blockers such as doxazosin and prazosin, calcium antagonists such as amlodipine and nifedipine, angiotensin-converting enzyme inhibitors such as captopril and imidapril, angiotensin II receptor antagonists such as losartan, candesartan, and valsartan, β blockers such as atenolol, and diuretic drugs such as furosemide.

Examples of prophylactics or therapeutic agents for diabetes mellitus include insulin sensitizing agents such as pioglitazone, troglitazone, and rosiglitazone, insulin secretion promoters such as tolbutamide, chlorpropamide, tolazamide, acetohexamide, glyclopyramide, glibenclamide, gliclazide, glimepiride, repaglinide, and nateglinide, biguanides such as metformin and buformin, α-glucosidase inhibitors such as insulin, acarbose, voglibose, miglitor, and emiglitate, β3 adrenoceptor agonists such as SR-58611-A, SB-226552, and AZ40140, ergoset, pramlintide, leptin, and BAY-27-9955.

Examples of prophylactics or therapeutic agents for hyperlipemia include HMG-CoA reductase inhibitors such as mevalotin and atorvastatin, anion exchange resins such as cholestyramine, the fibrate class of drugs such as bezafibrate, nicotinic acid derivatives such as niceritrol, and probucol.

Examples of coronary vasodilators include nitrates such as nitroglycerin.

### Brief Description of Drawings

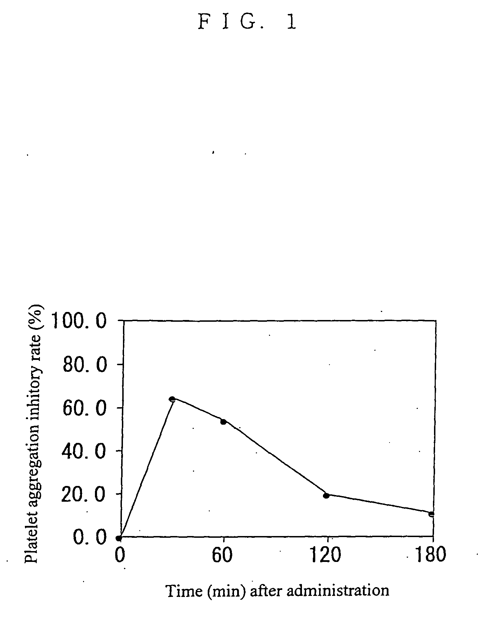

Figure 1 shows the results obtained after oral administration of a benzomorpholine derivative of the present invention.

This specification includes part or all of the contents as disclosed in the specification and/or drawings of Japanese Patent Application No. 2002-355544, which is a priority document of the present application.

### Best Mode for Carrying Out the Invention

The present invention will now be specifically described by way of reference examples and examples.

### (Reference Example 1) Mesylation Reaction:

To a solution of methyl 4-(2-hydroxyethyl)-3,4-dihydro-2H-1,4-benzoxazine-8-yloxyacetate (15.7 g, 58.7 mmol) and triethylamine (29.5 ml, 211.7 mmol) in methylene chloride (360 ml), mesyl chloride (4.6 ml, 58.3 mmol) was added at 0 °C, followed by stirring for one hour. The reaction solution was added to 5% aqueous citric acid, followed by ethyl acetate extraction. Organic layers were washed with water and brine, dried over MgSO₄, and then concentrated to obtain a mesylated product (17.8 g, 51.5 mmol). The mesylated product required no purification and was directly used for the next reaction.

### (Example 1) Condensation Reaction (General Procedure):

A solution of benzanilide (15 mmol) in DMF (30 ml) was added to NaH (15 mmol), followed by stirring at room temperature for 30 minutes.

Subsequently, a solution of methyl 4-(2-mesyloxyethyl)-3,4-dihydro-2H-1,4-benzoxazine-8-yloxyacetate (12 mmol) obtained in Reference example 1 in DMF (15 ml) was added, followed by stirring at 80°C for 5 hours. The solvent was removed under reduced pressure and then the residue was added to 5% citric acid, followed by ethyl acetate extraction. Organic layers were washed with water and brine, dried over MgSO₄, and then concentrated. The residue was purified by column chromatography (silica gel, eluent; AcOEt/n-hexane = 1:1) and recrystallization (AcOEt/n-hexane) to give a target methyl ester.

Subsequently, to a solution of the methyl ester in ethanol (10 ml) and THF (30 ml), 2.0 N aqueous sodium hydroxide (1.2 eq) was added, followed by stirring at room temperature for one hour. The solvent was removed under reduced pressure. The residue was added to an aqueous 1N-HCl, followed by ethyl acetate extraction. Organic layers were washed with water and brine, dried over MgSO₄, and then concentrated. The residue was recrystallized from AcOEt/n-hexane to obtain a target product.

Various derivatives were synthesized by similar reactions and procedures. Raw materials and products are shown in Tables 8 to 16 and spectrum data are shown in Tables 17 to 25.

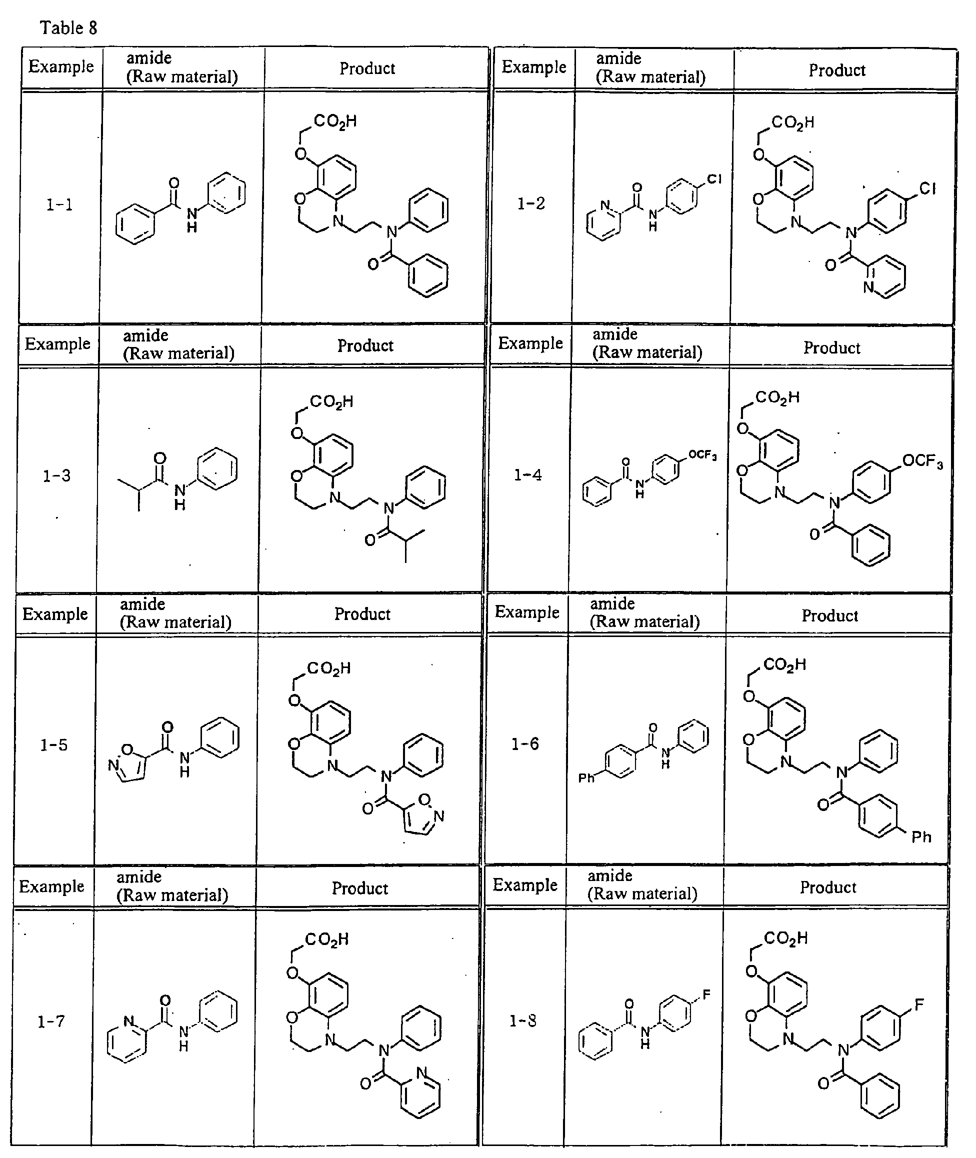

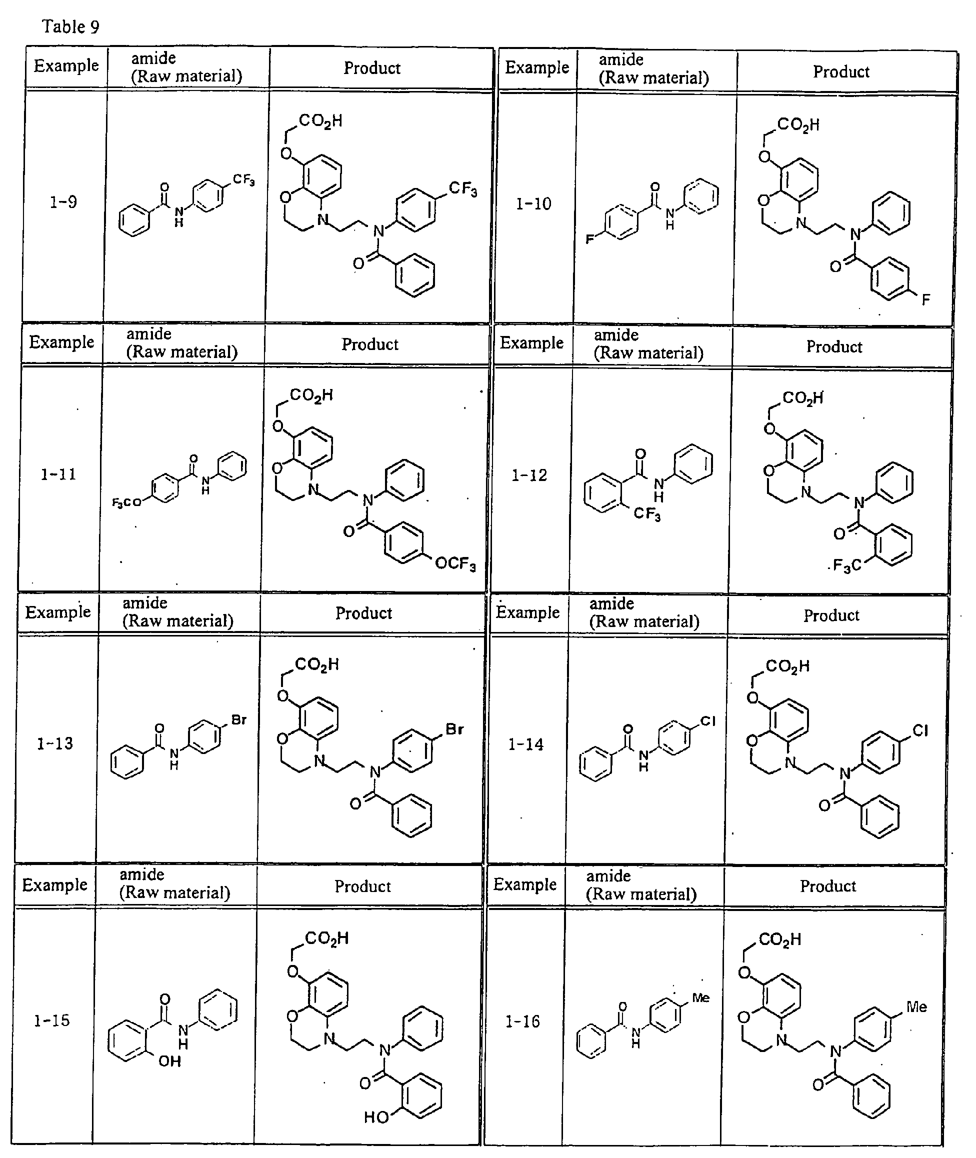

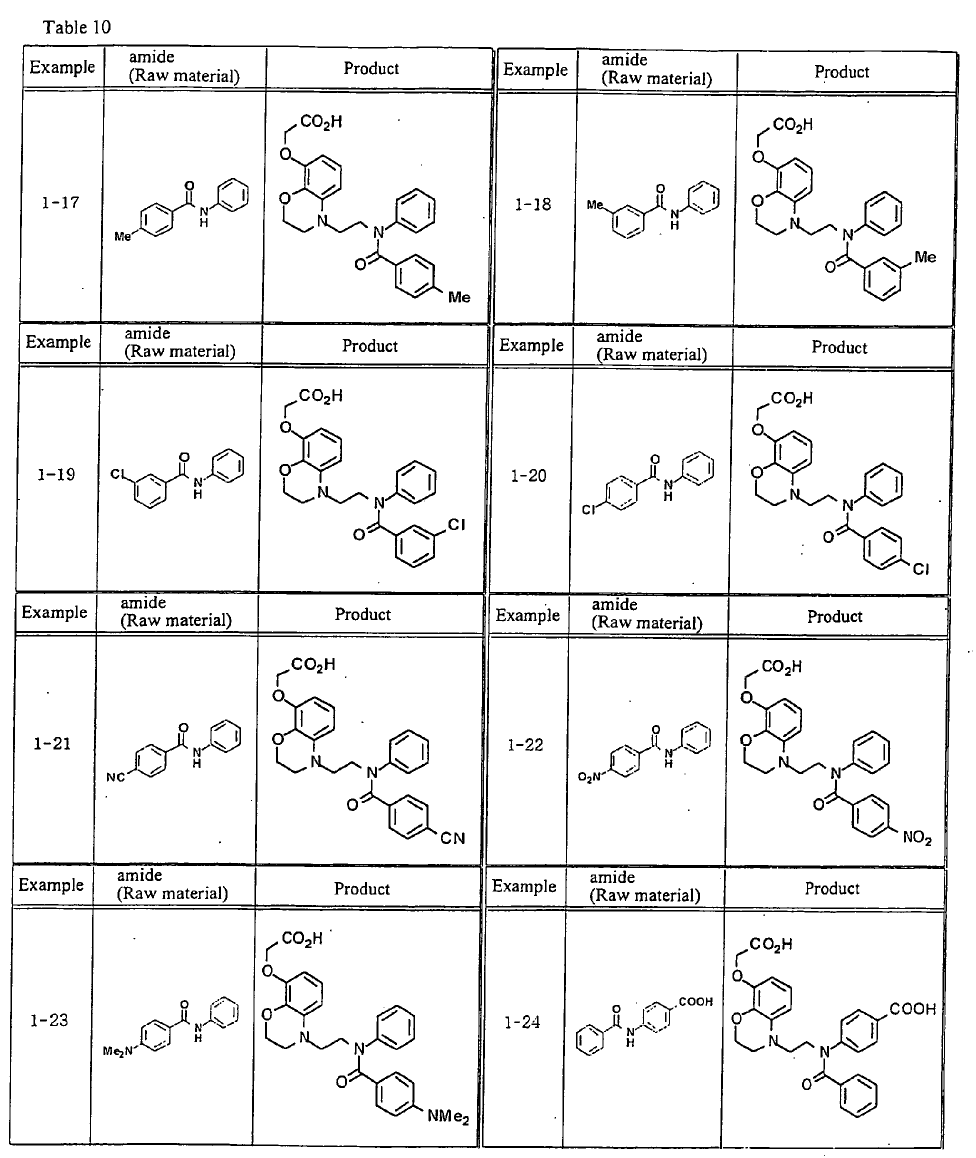

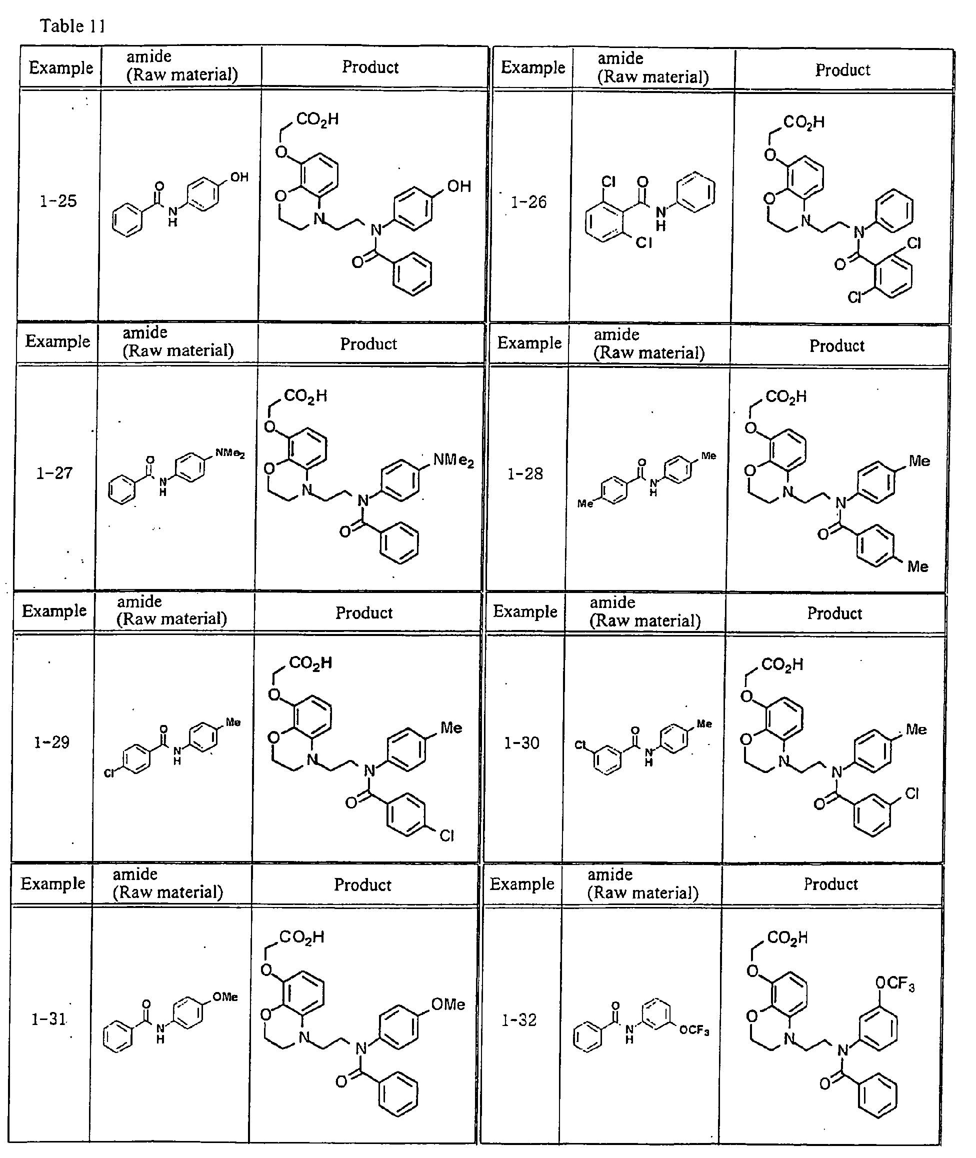

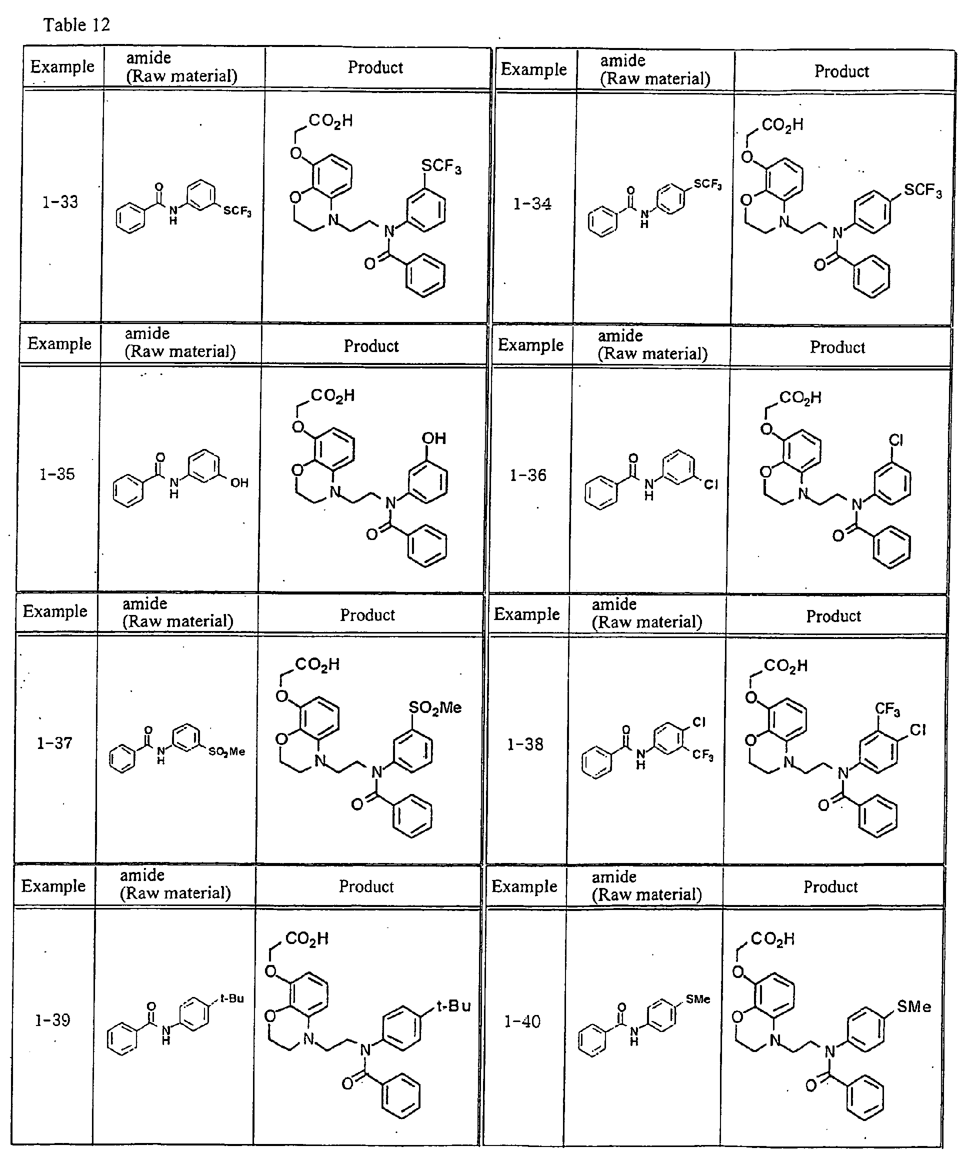

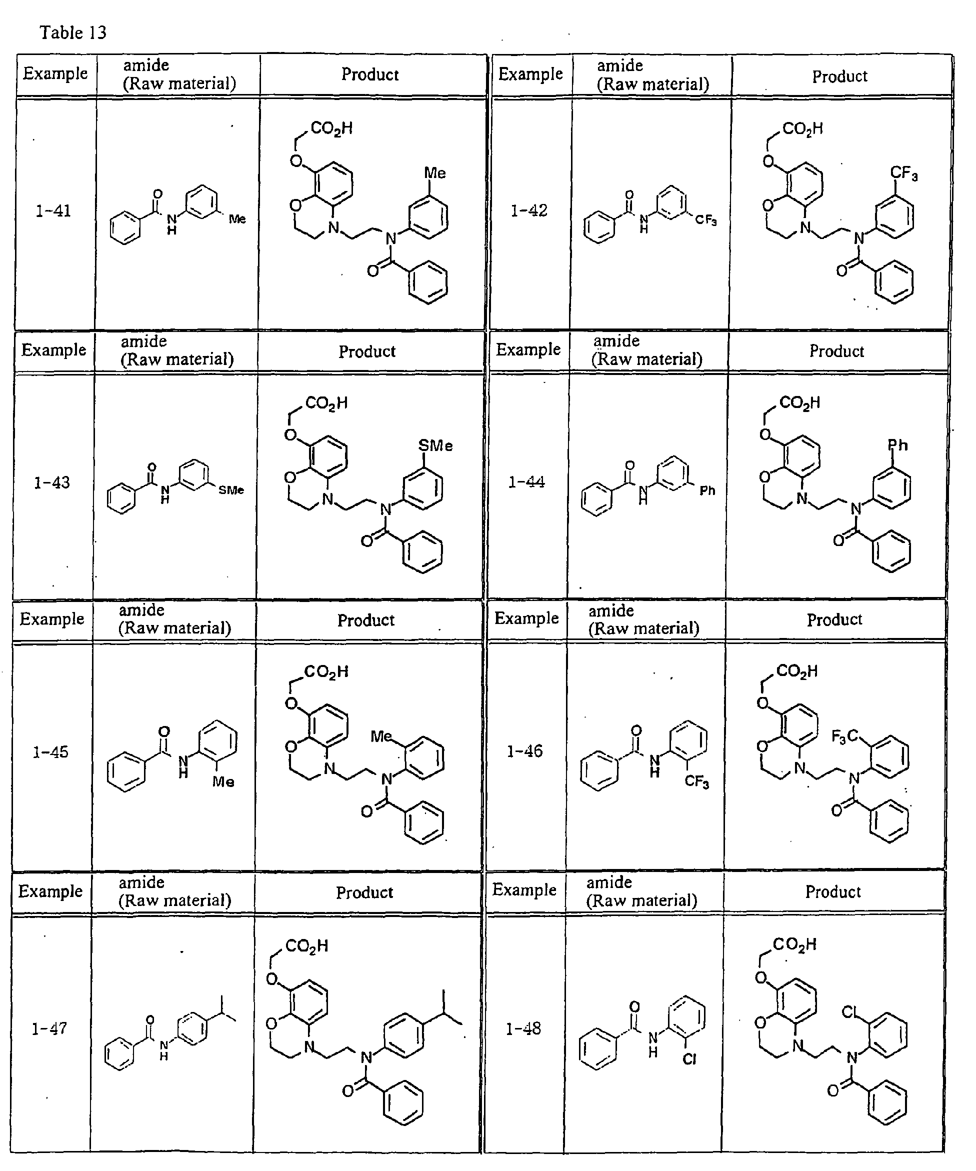

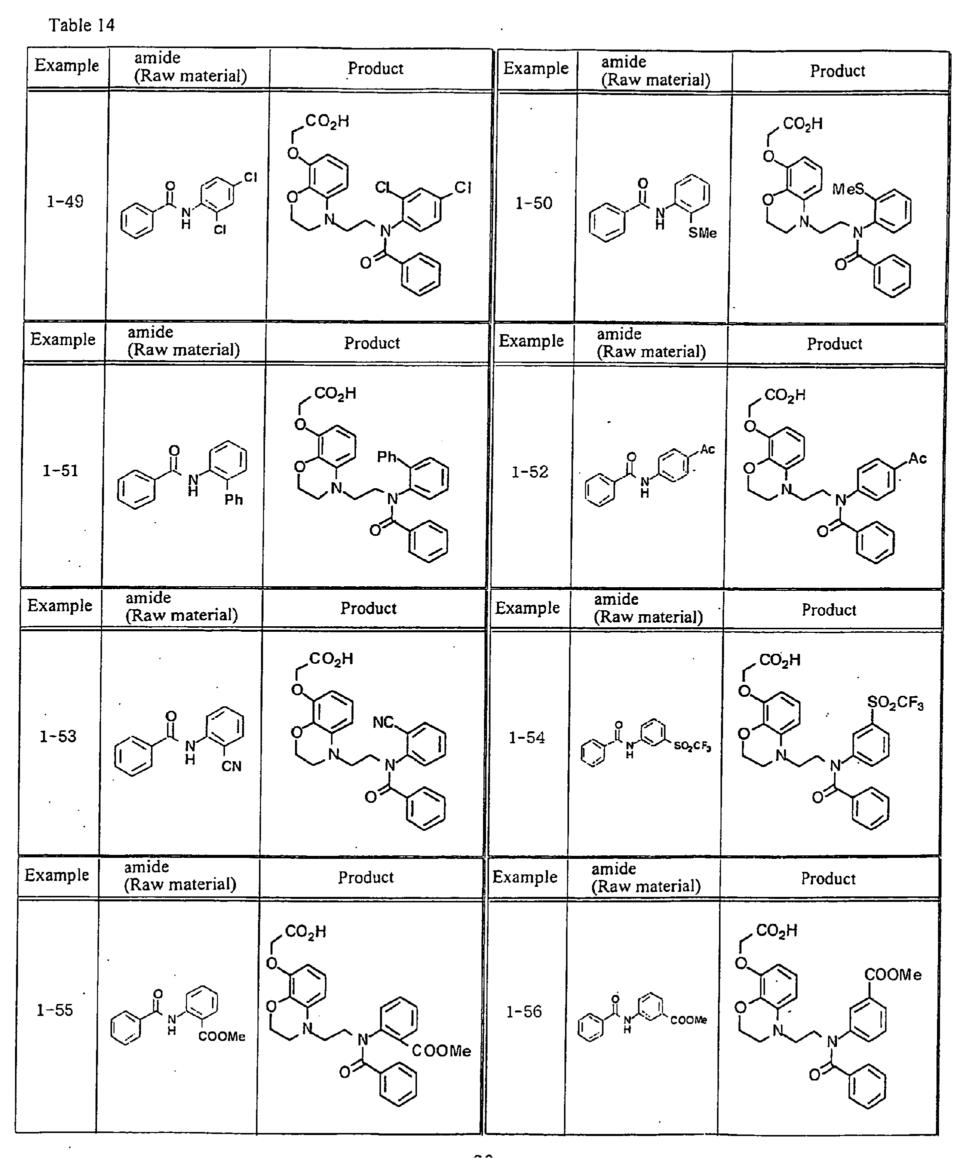

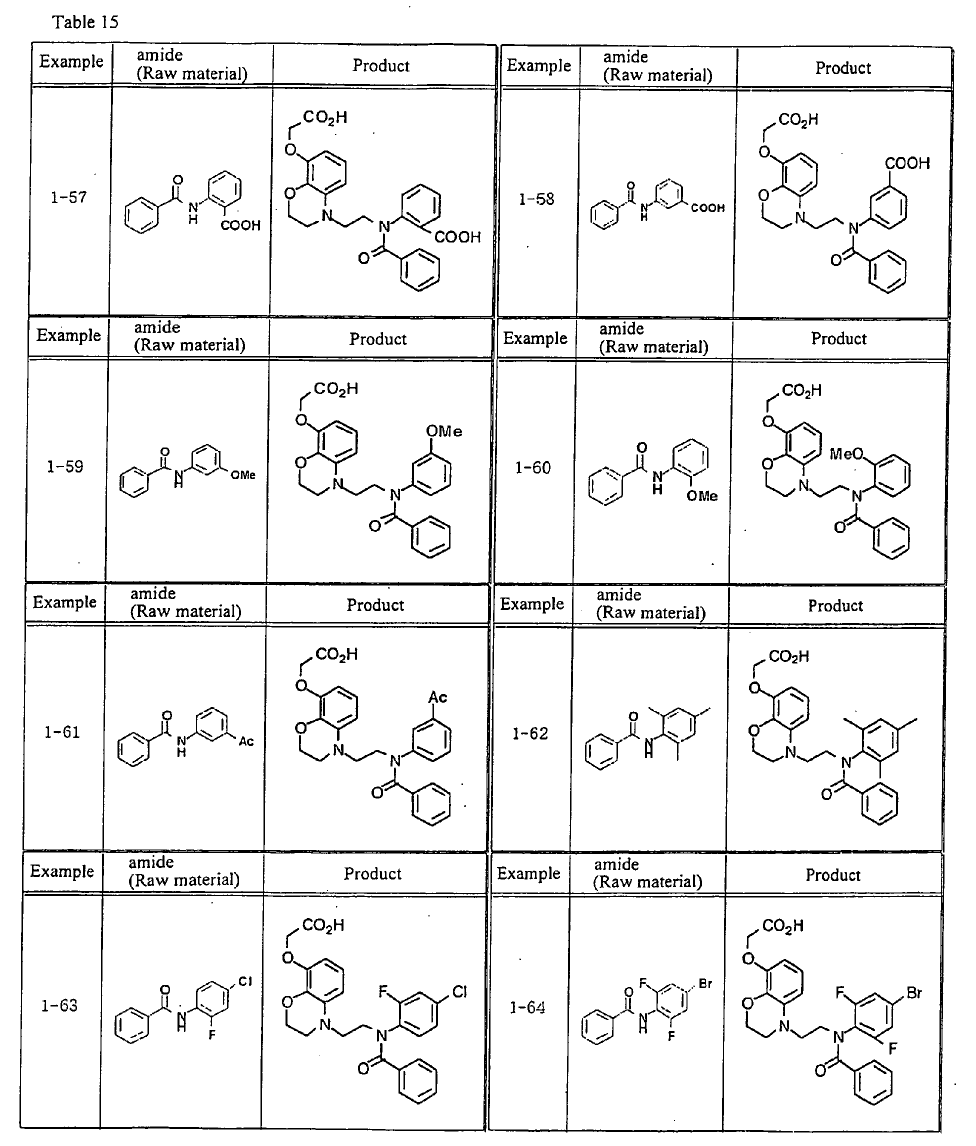

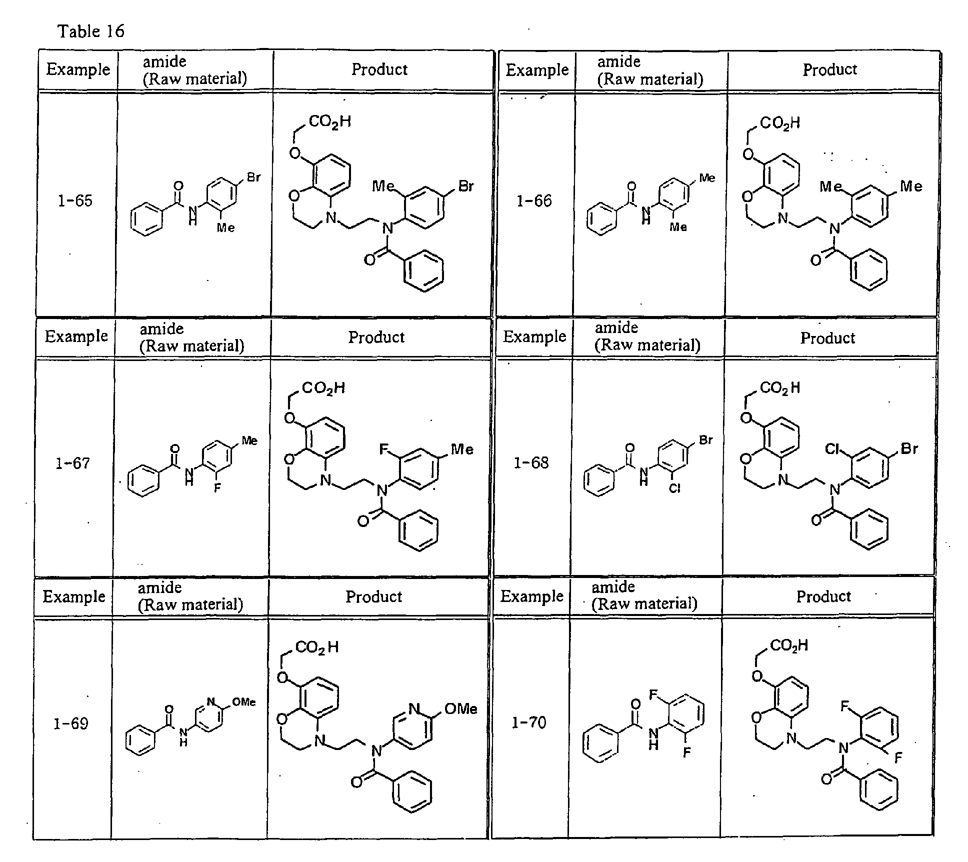

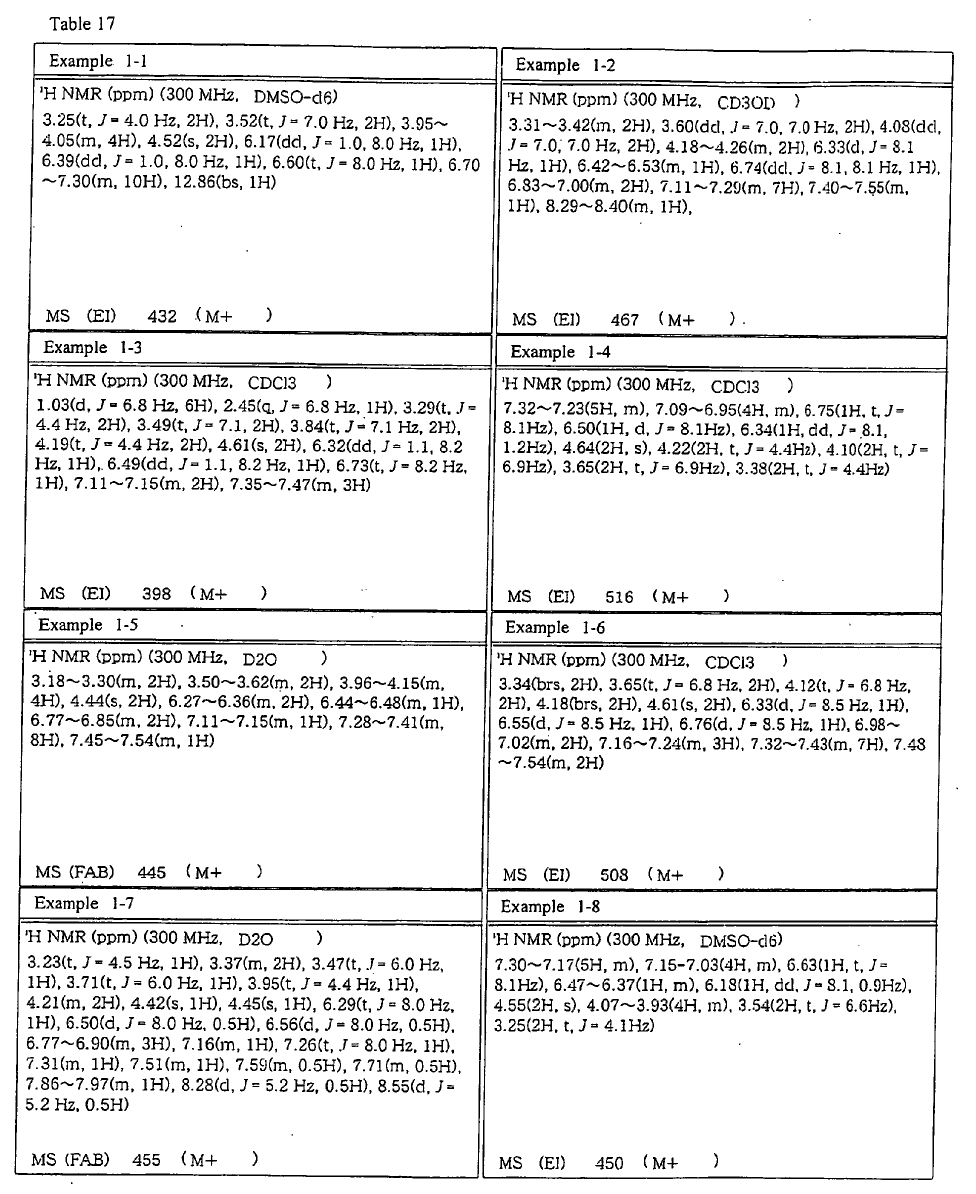

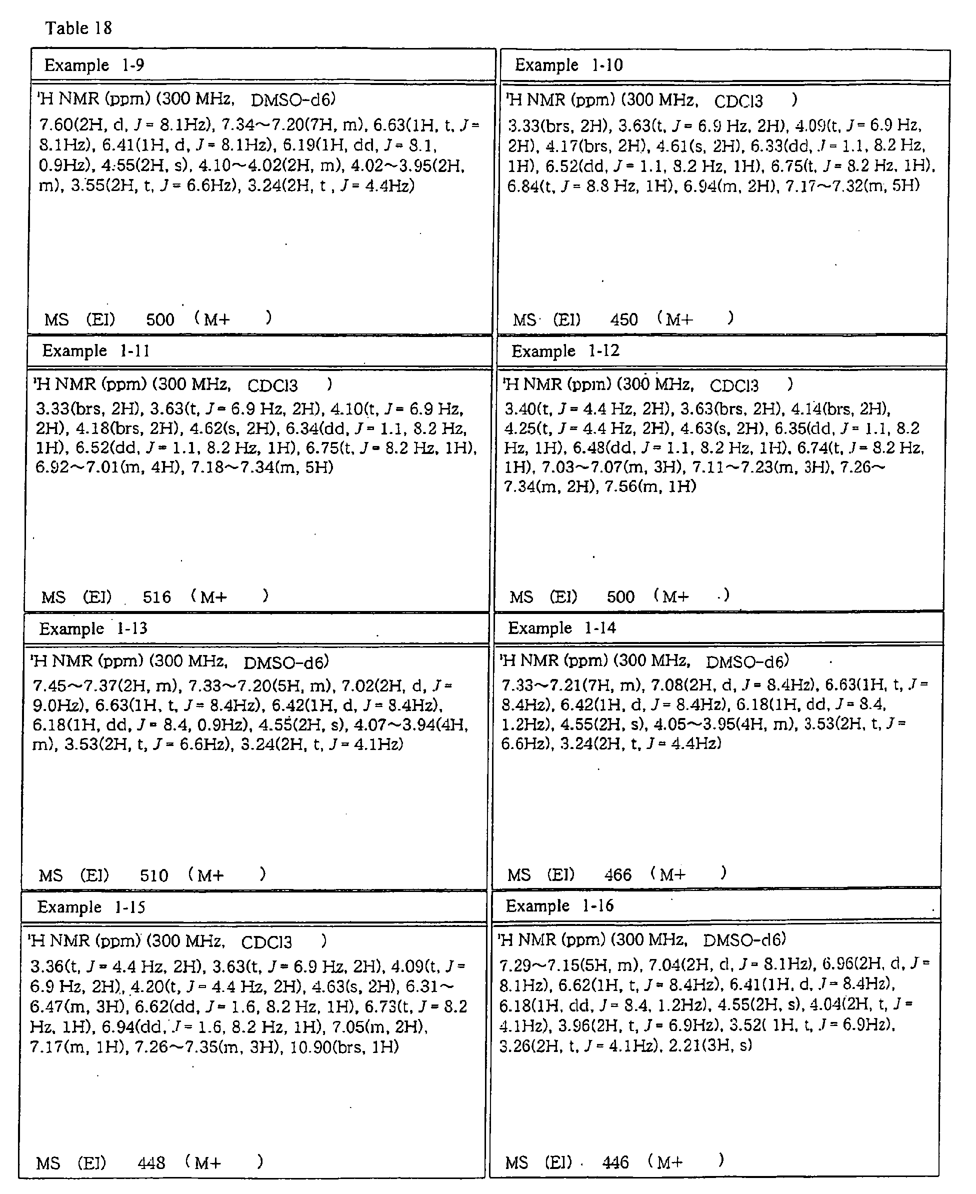

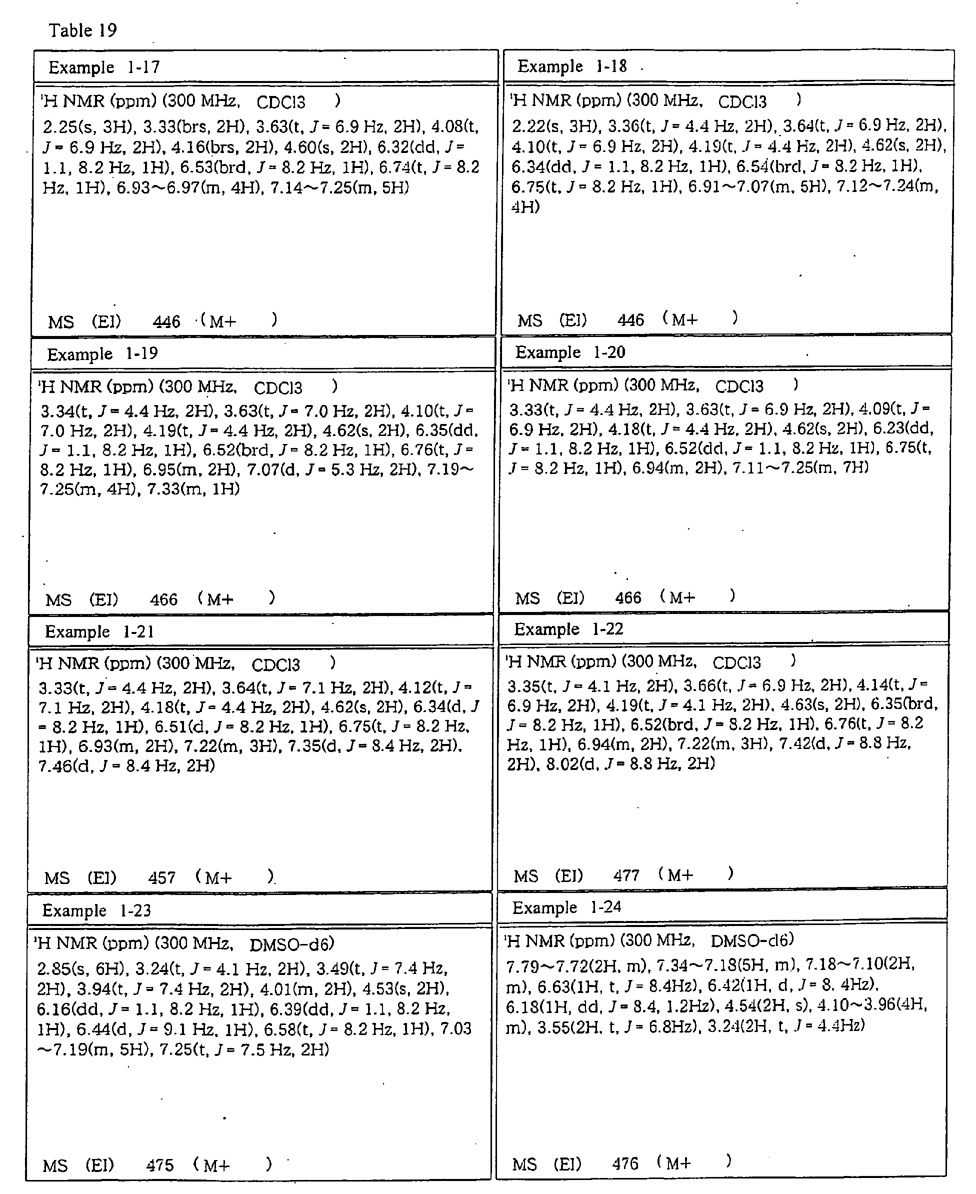

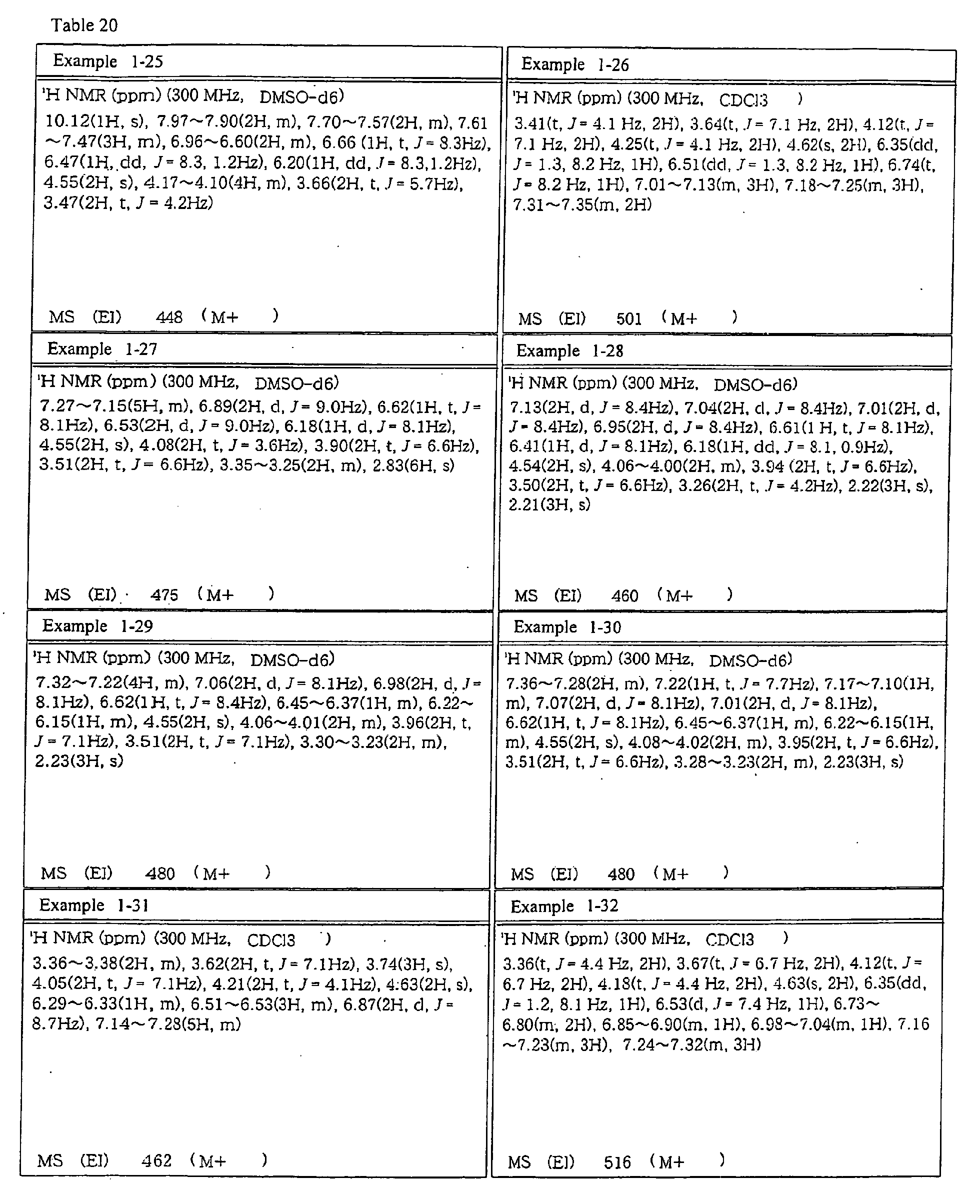

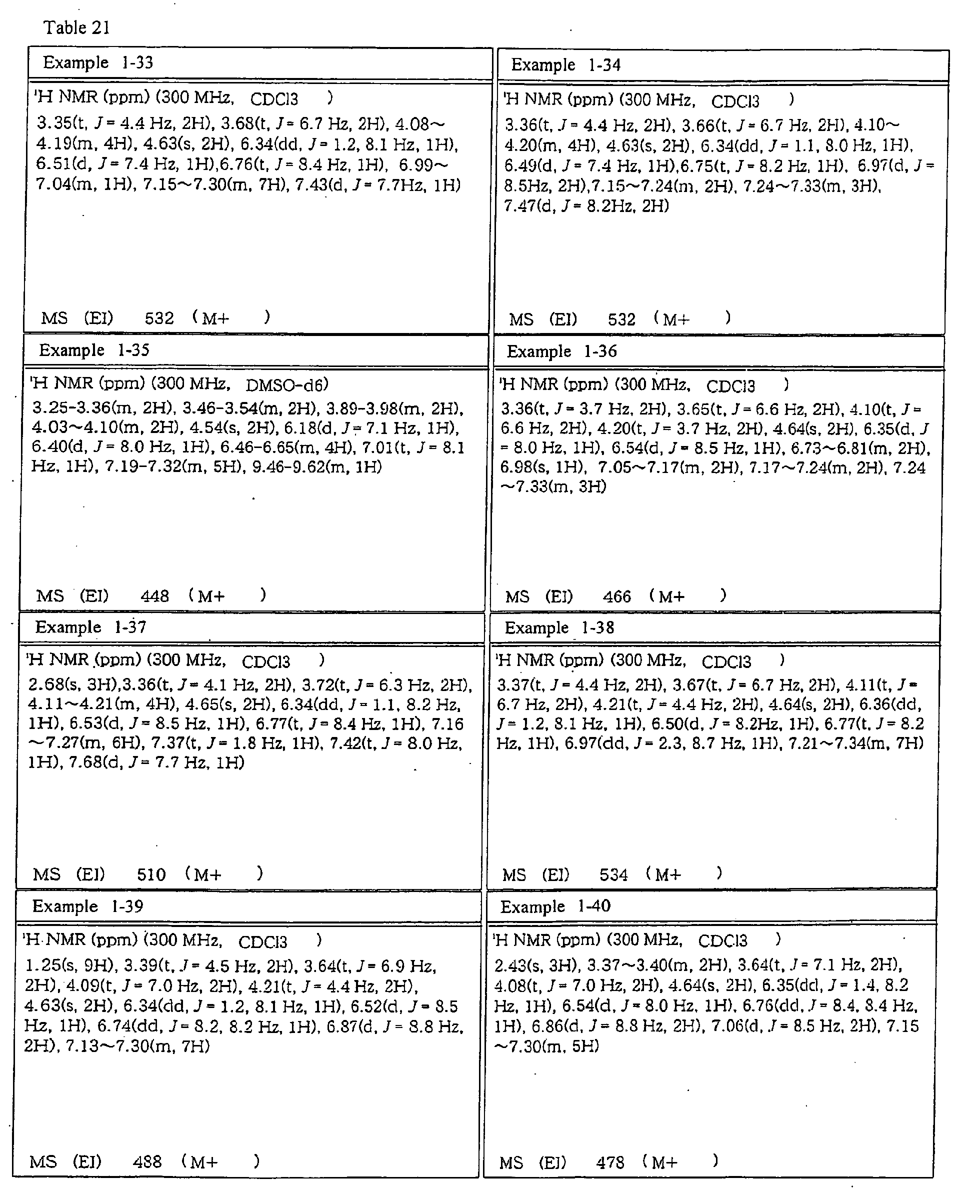

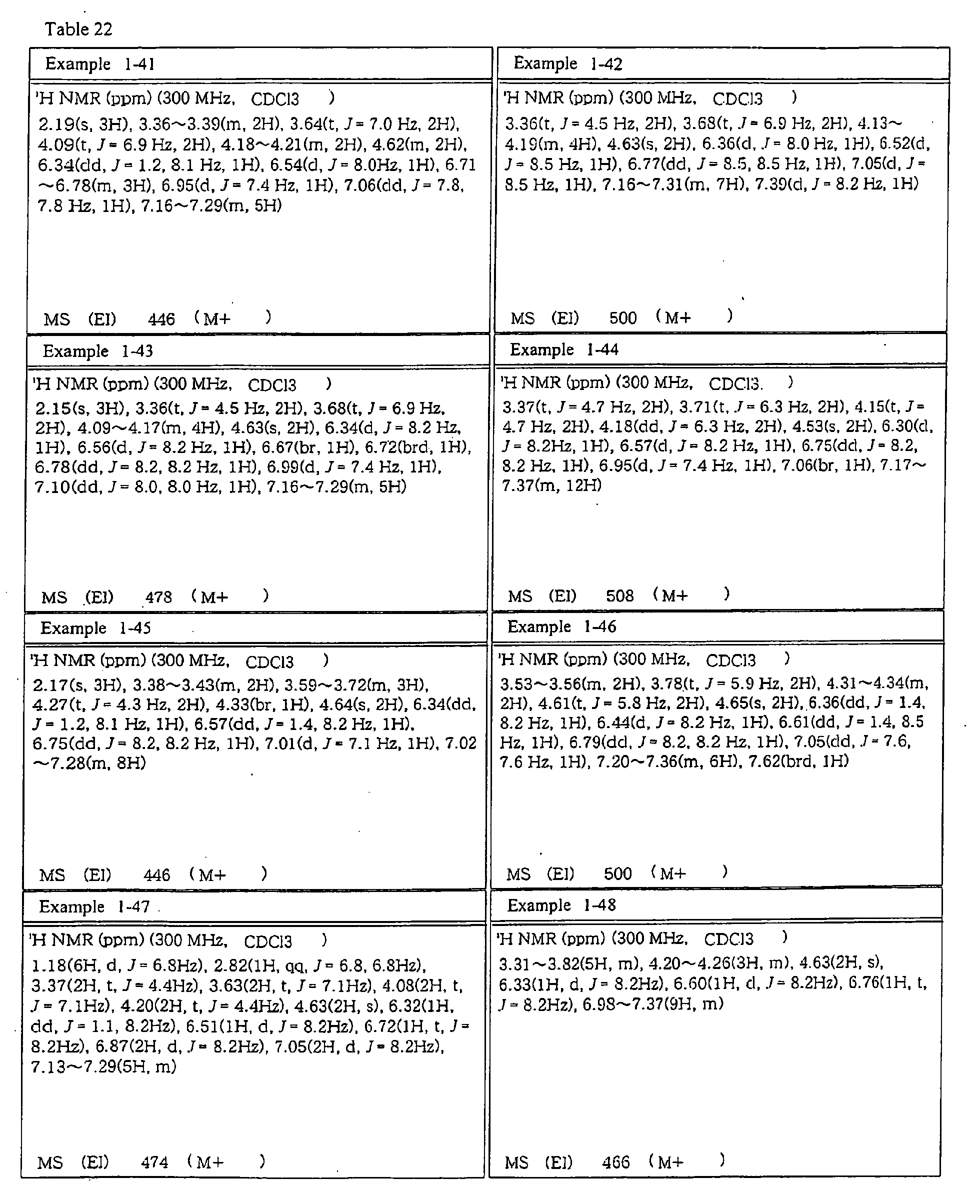

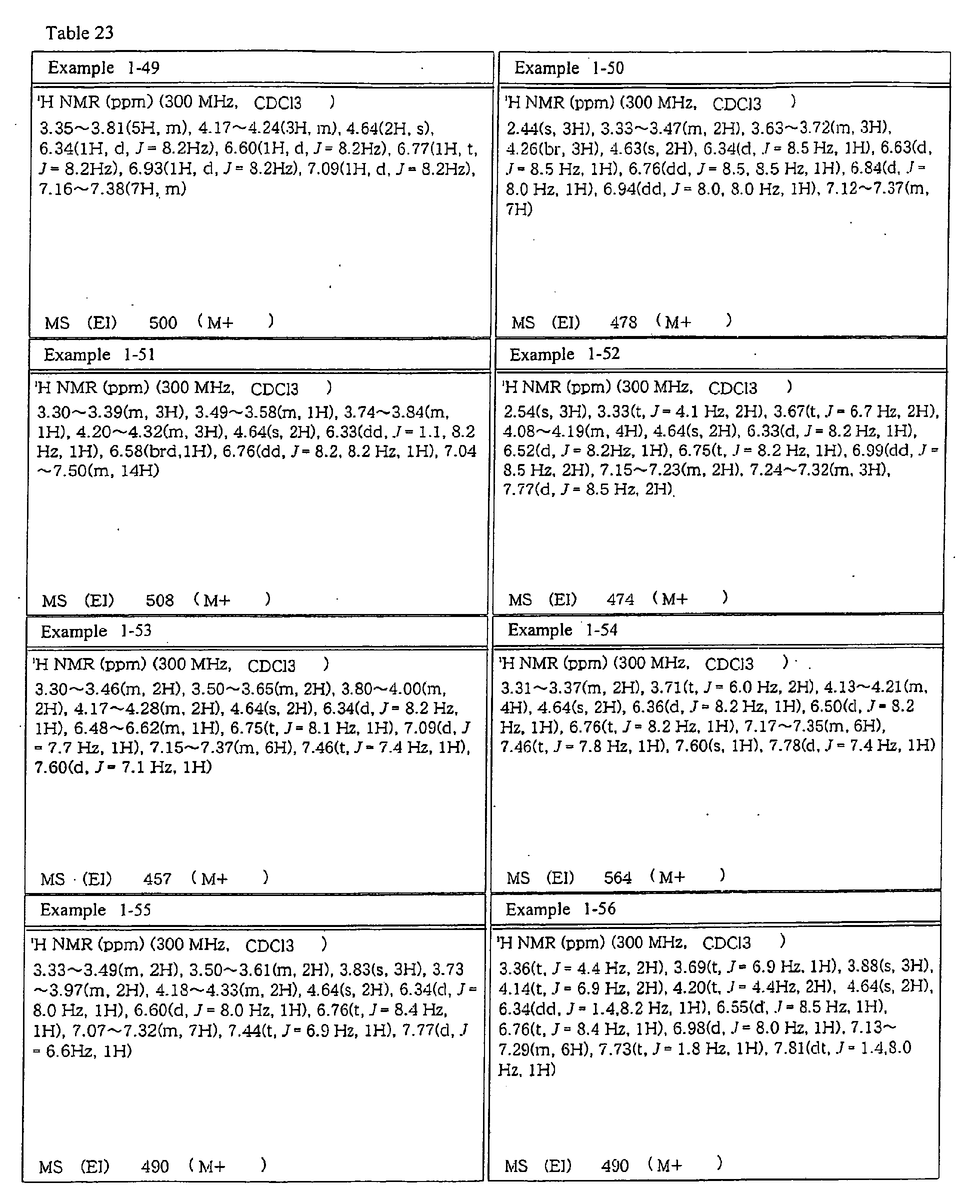

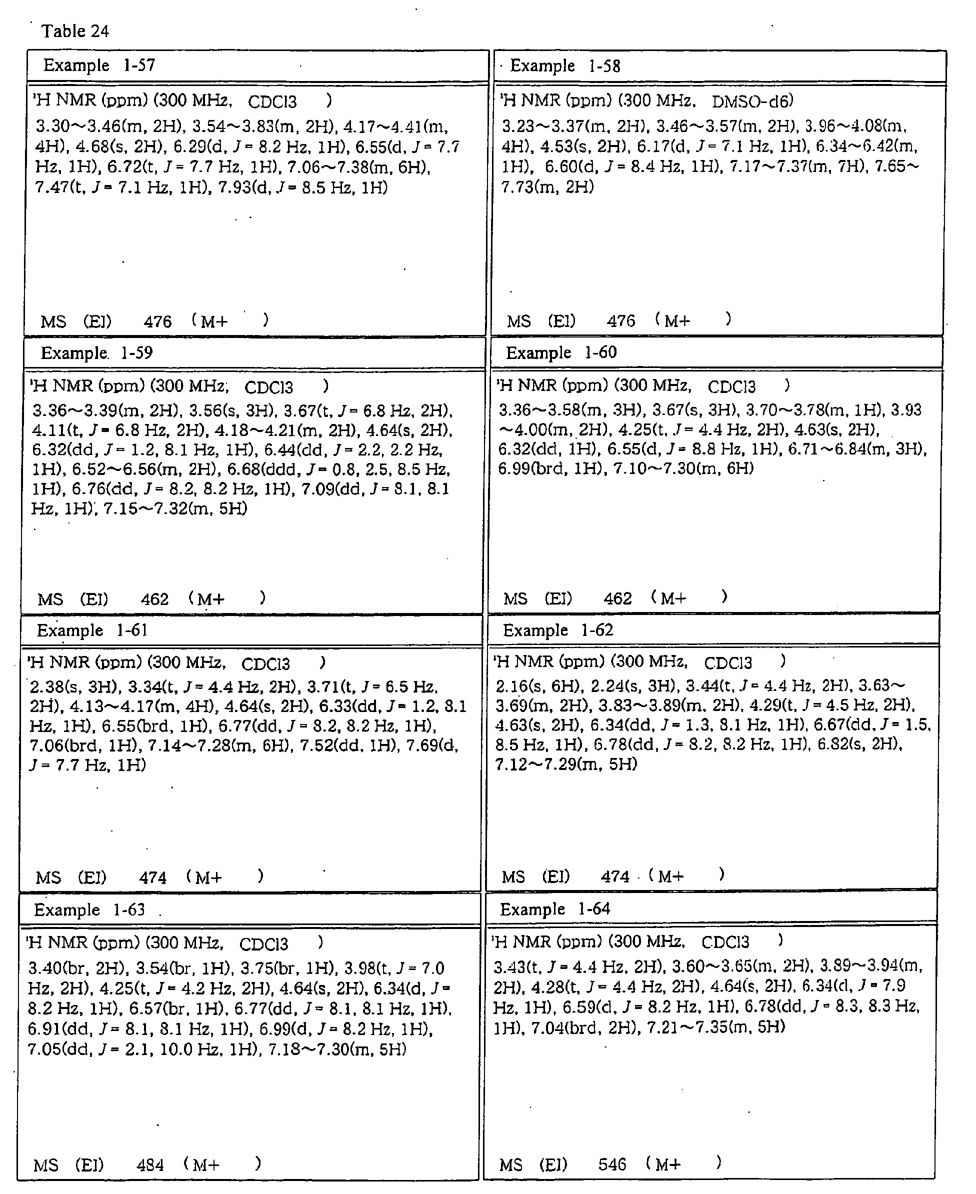

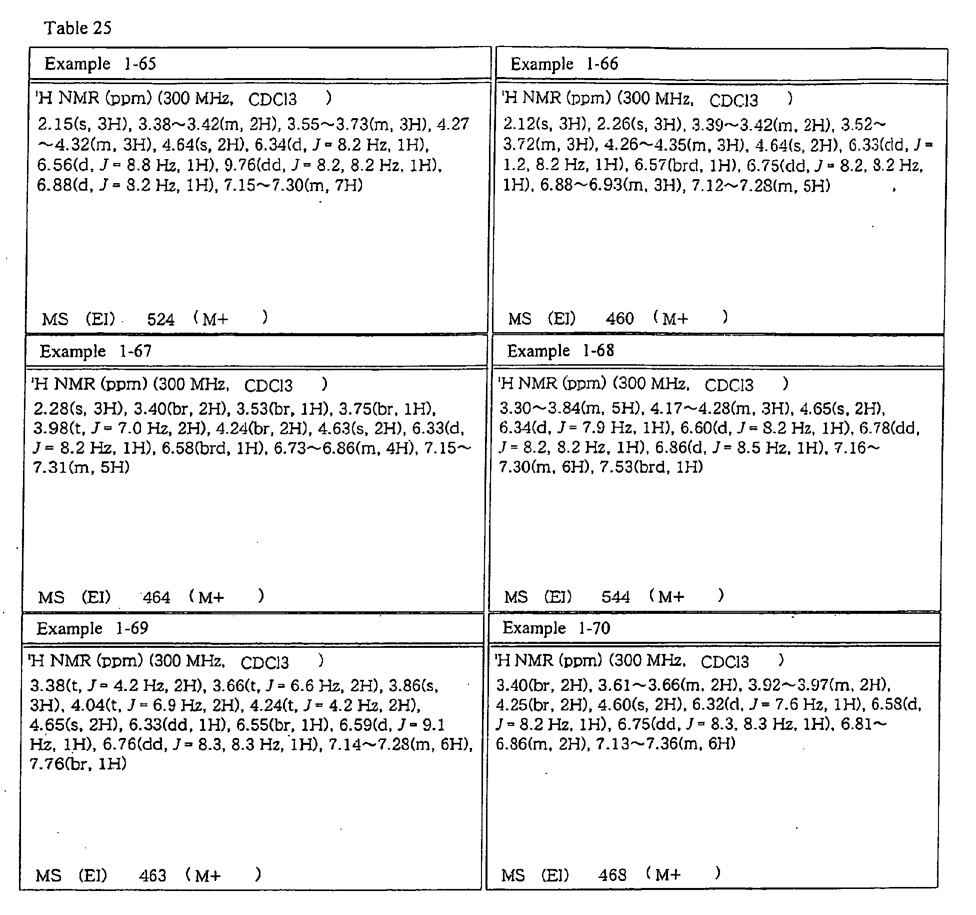

### (Reference example 2) N-alkylation Reaction:

4-Bromoaniline (440 mg, 2.6 mmol) was added to a solution of the mesylated product (0.52 mmol) obtained in Reference example 1 in acetonitrile (10 ml), followed by heating under reflux for 48 hours. Saturated aqueous NaHCO₃ was added to the reaction mixture, followed by ethyl acetate extraction. Organic layers were washed with water and brine, dried over MgSO₄, and then concentrated. The residue was purified by column chromatography (silica gel, eluent; AcOEt/n-hexane = 1:1), so that N-alkylated product (0.45 mmol) was obtained in the form of white crystal (melting point between 76°C and 79°C).

¹H NMR data of the obtained N-alkylated product are shown below.
¹H NMR (300 MHz, CDCl₃) δ 3.30-3.40 (m, 4H), 3.48 (t, J = 2.9 Hz, 2H), 3.80 (s, 3H), 4.28 (dd, J = 2.0, 2.0 Hz, 2H), 4.68 (s, 2H), 6.27 (dd, J = 8.2, 1.2 Hz, 1H), 6.43 (dd, J = 8.2, 1.2 Hz, 1H), 6.50 (d, J = 8.9 Hz, 2H), 6.73 (t, J = 8.2Hz, 2H), 7.26 (d, J = 8.9 Hz, 2H)

### (Example 2) Amidation Reaction:

3-Fluorobenzoyl chloride (1.3 mmol) was added to a solution of the N-alkylated product (0.92 mmol) obtained in Reference example 2 and triethylamine (1.8 mmol) in THF (6 ml), followed by stirring at room temperature for 3 hours. The reaction mixture was added to water, followed by ethyl acetate extraction. Organic layers were washed with water and brine, dried over MgSO₄, and then concentrated. The residue was recrystallized from AcOEt/n-hexane to obtain a target methyl ester.

Subsequently, 2.0 N aqueous sodium hydroxide (3.0 eq) was added to a solution of the methyl ester in ethanol (5 ml) and THF (5 ml), followed by stirring at room temperature for 2 hours. The solvent was removed under reduced pressure, and then the residue was added to aqueous 1N-HCl, followed by ethyl acetate extraction. Organic layers were washed with water and brine, dried over MgSO₄, and then concentrated. The residue was recrystallized from AcOEt/n-hexane to obtain a target product.

Various derivatives were synthesized by similar reactions and procedures. Raw materials and products are shown in Tables 26 to 29 and spectrum data are shown in Tables 30 to 33.

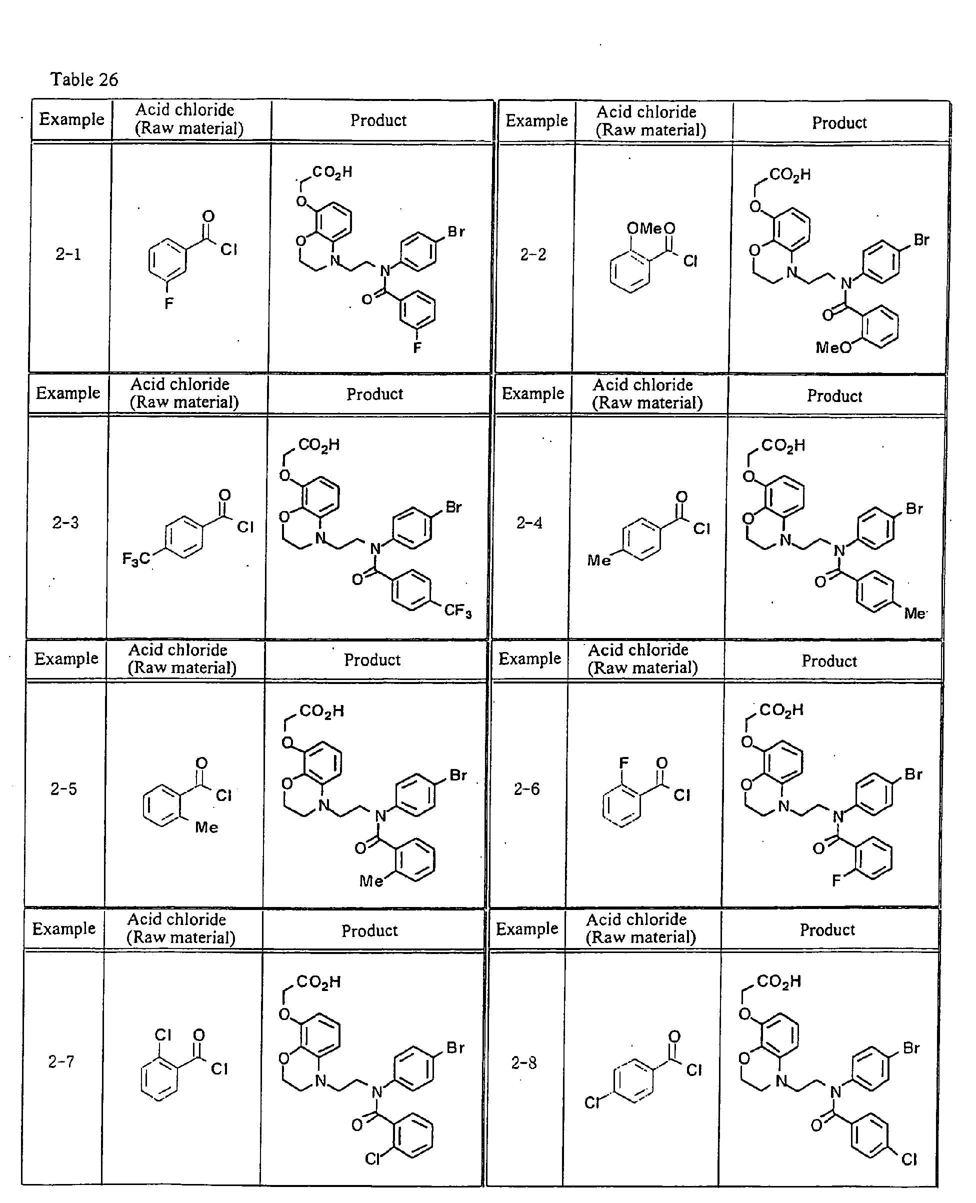

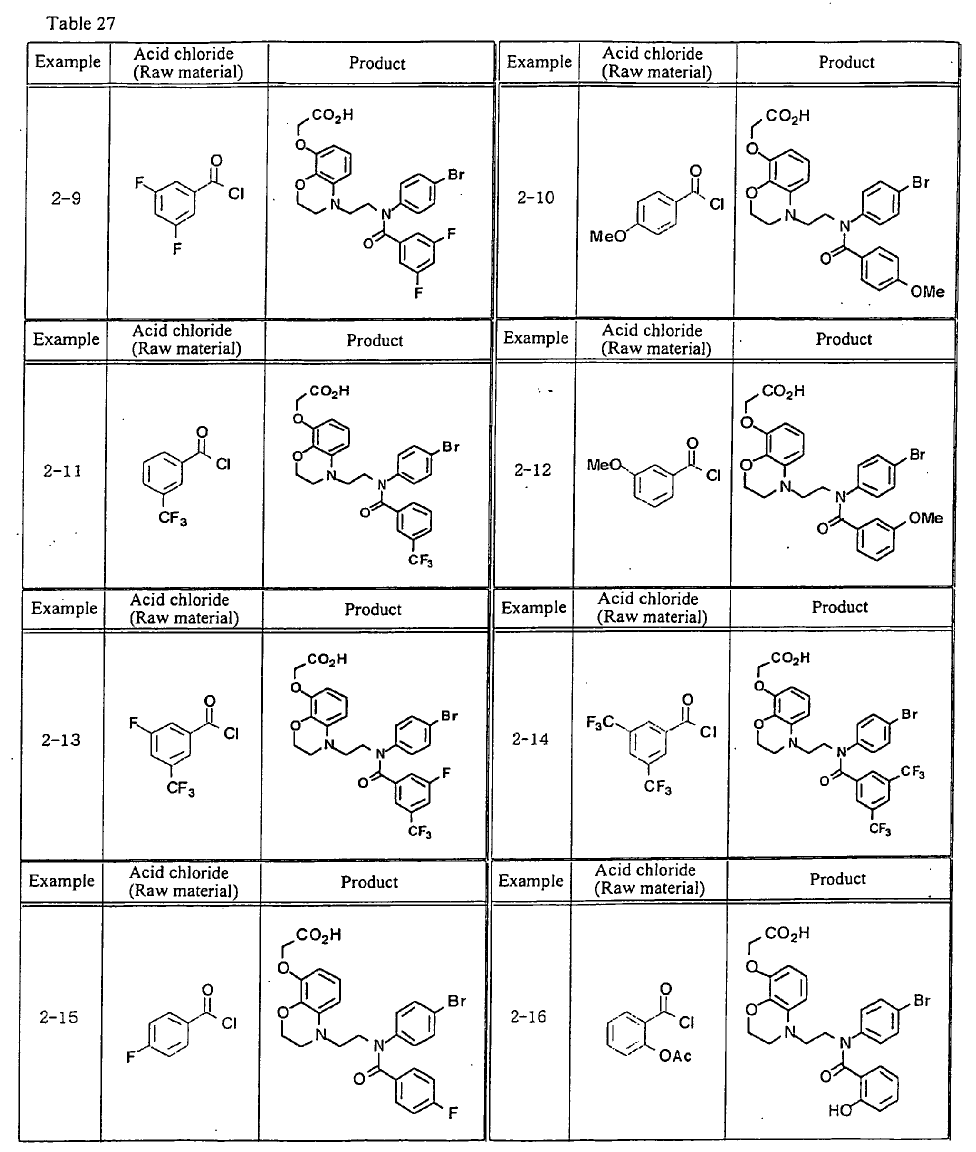

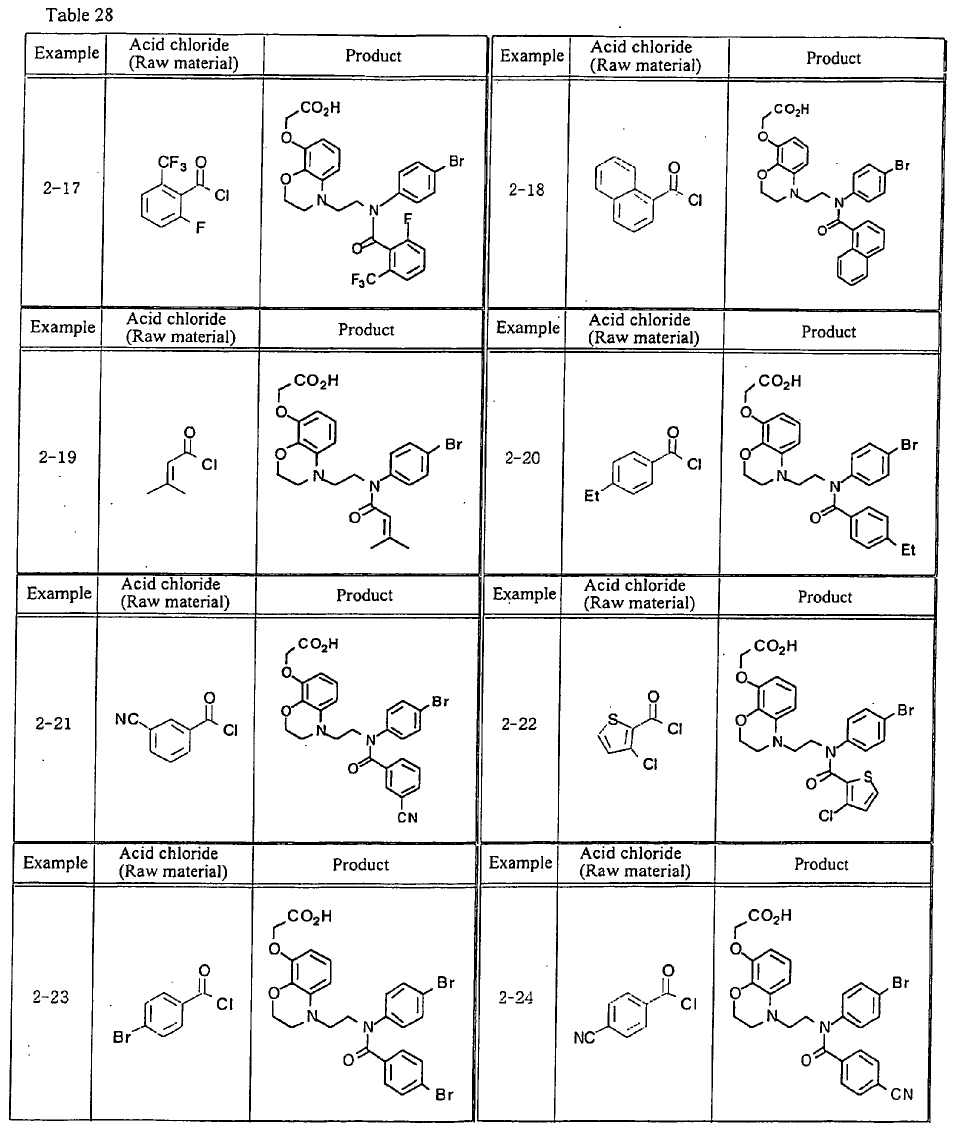

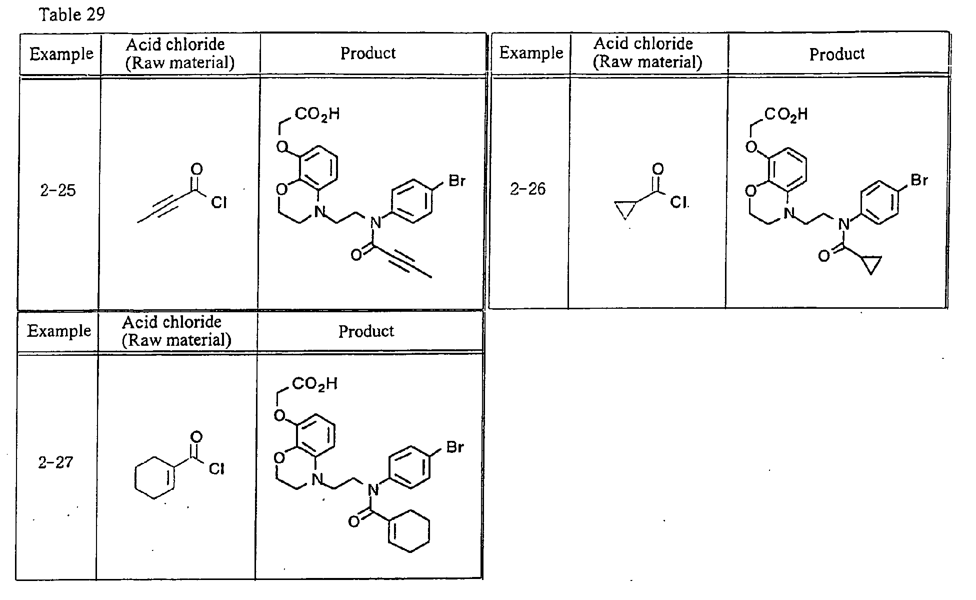

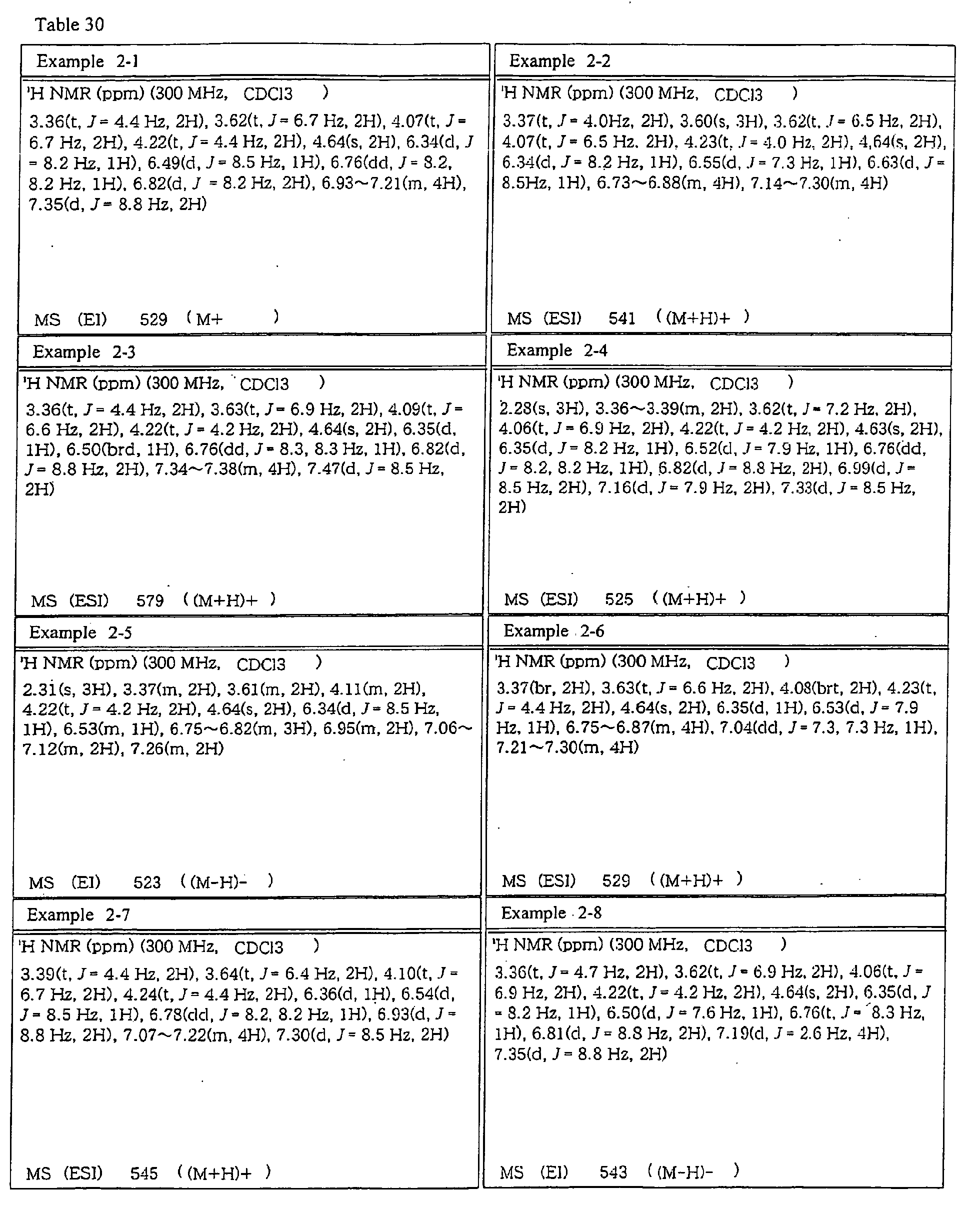

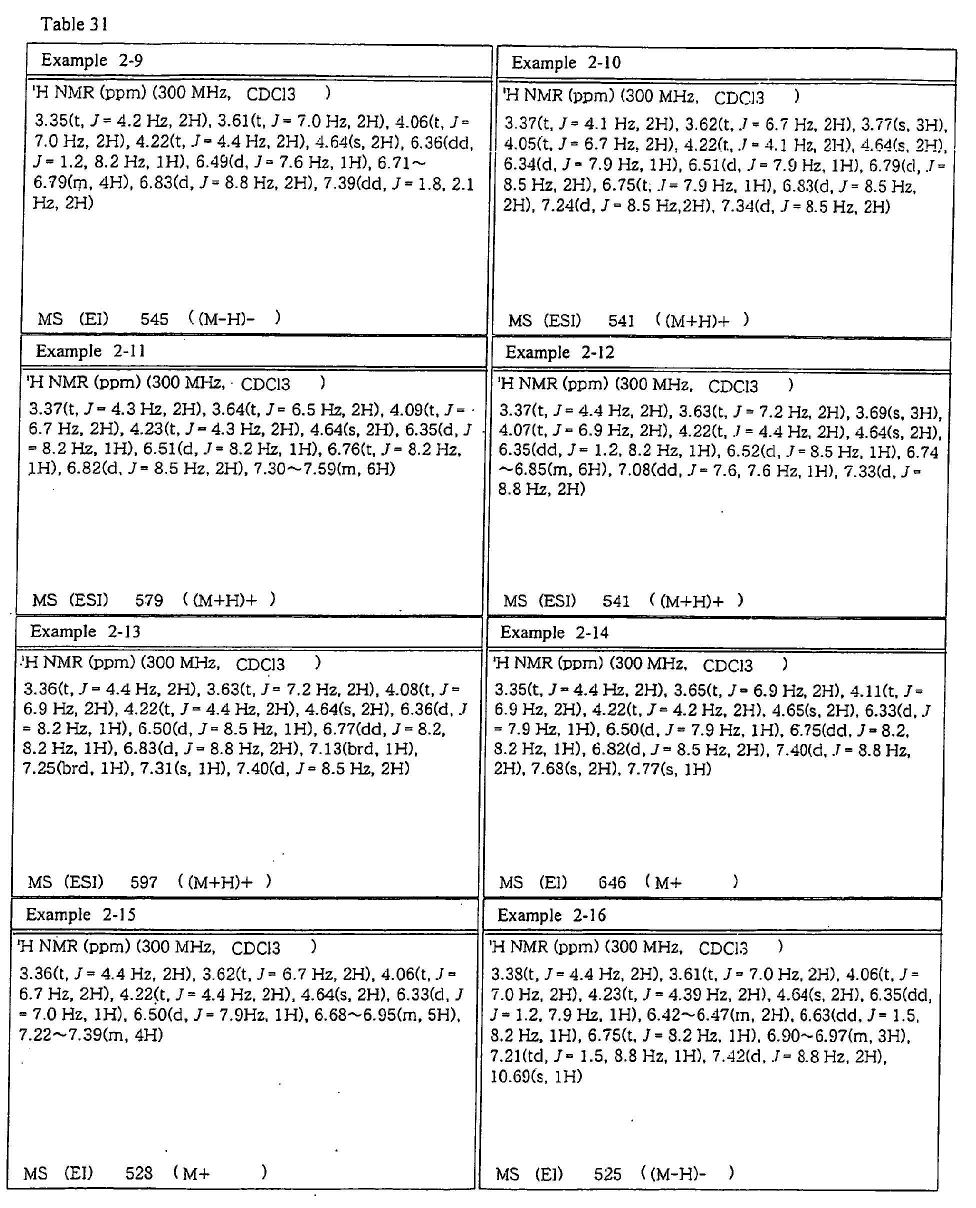

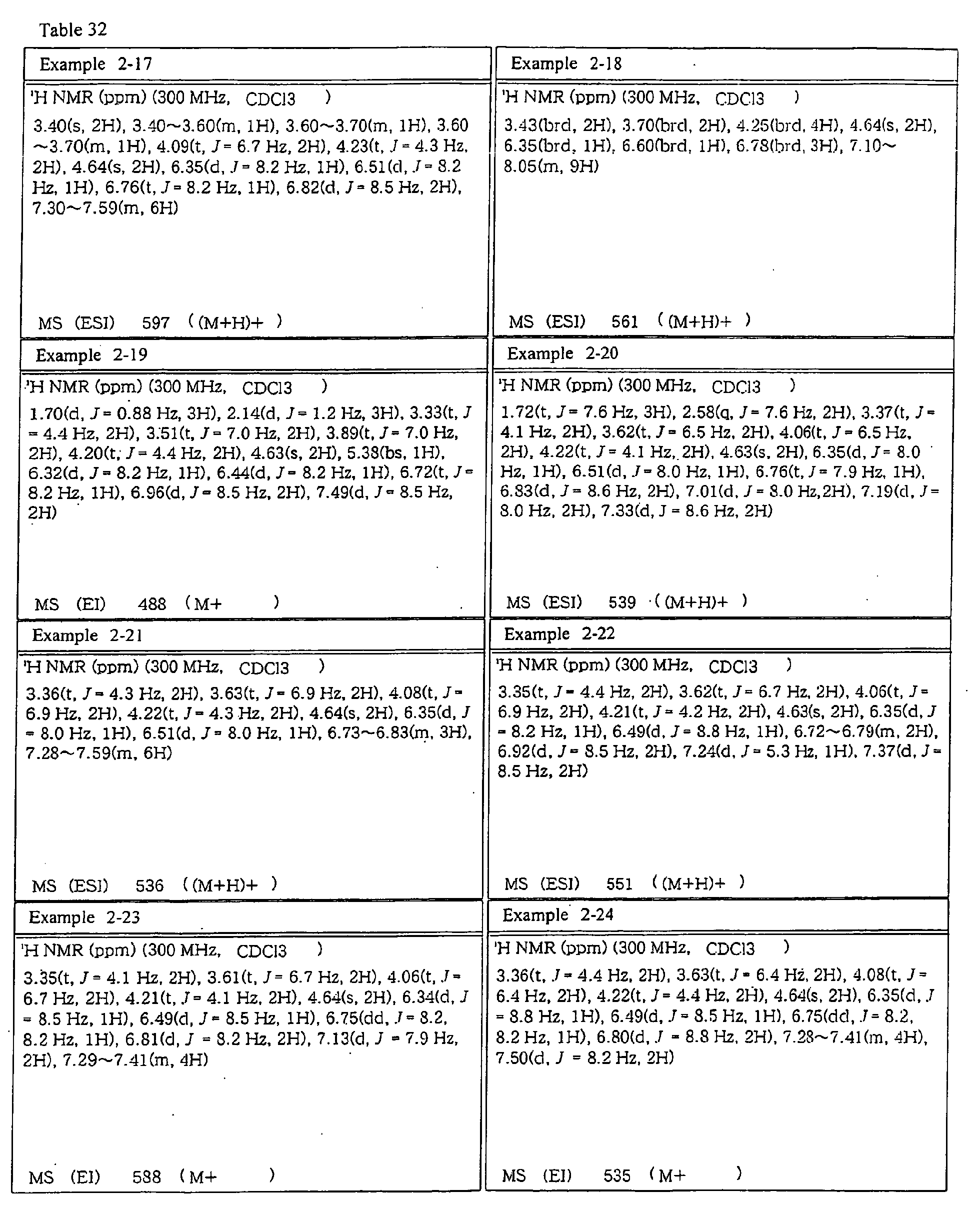

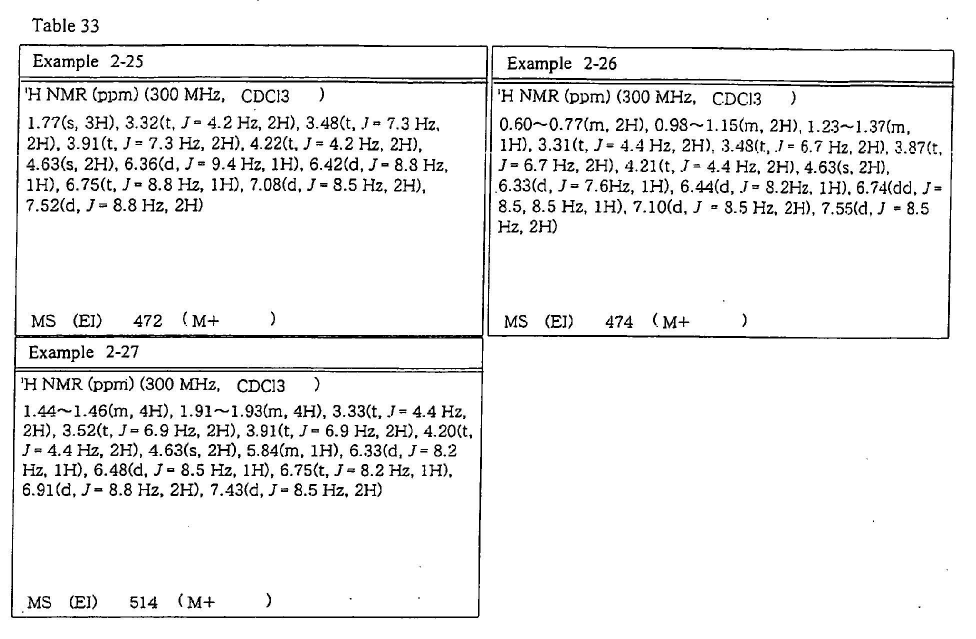

### (Example 3) Human platelet aggregation inhibitory test:

Blood collected from human median cubital vein was centrifuged at 800 rpm for 10 minutes. Upper portion of the resultant was collected as platelet rich plasma (PRP). PRP was dispensed to small test tubes, and 5 µM of ADP was added thereto to induce platelet aggregation. The degree of platelet aggregation was measured as a change in turbidity using an aggregometer (Hematracer 1, Nikobioscience). Each compound was added 1 minute before ADP addition, and calculation was carried out using as an IC₅₀ value a concentration that inhibits aggregation by 50%.

The results of evaluating the activities of the compounds of the present invention by the methods are summarized in Table 34. As a result, the benzomorpholine derivatives of the present invention were revealed to have a stronger inhibitory effect of platelet aggregation compared with that of the compound used in Example 21 in Patent document 1.

**Table34**

| Example No. of Compound | Platelet Aggregation Inhibitory Effect IC50 (nM) |
|---|---|
| 1-31 | 31 |
| 1-14 | 13 |
| 1-16 | 5.3 |
| 1-28 | 5.9 |
| 1-31 | 14 |
| 1-40 | 14 |
| 1-49 | 18 |
| 1-63 | 8.8 |
| 2-4 | 27 |
| 2-27 | 55 |
| Patent Document * Compound of Example 21 | 1,800 |

| | |
|---|---|
| *WO00/07992 | |

### (Example 4) Monkey Platelet Aggregation Inhibitory Test:

Male crab-eating monkeys (4 to 6 kg) were retained in monkey chairs. After 30 minutes of acclimatization, the compound of Example 1-13 was orally administered to the monkeys using catheters (16 Fr).

Blood was collected via saphenous veins before, and 30, 60, 120 and 180 minutes after administration of drug solutions using a syringe that had previously contained a 3.8% sodium citrate solution in a volume that was one-tenth of that of collected blood.

The collected blood was centrifuged at 1000 rpm for 10 minutes. Upper portions of the resultants were collected as platelet rich plasma (PRP). PRP was apportioned into small test tubes, and 10 µM of ADP was added thereto to induce platelet aggregation. The degree of platelet aggregation was measured as a change in turbidity using an aggregometer (Hematracer 1, Nikobioscience).

The results of evaluating activity of the benzomorpholine derivative of the present invention after oral administration thereof by the aforementioned methods are shown in Figure 1. As a result, the benzomorpholine derivative of the present invention was revealed to have a strong inhibitory effect of platelet aggregation also by oral administration thereof to monkeys.

All publications, patents, and patent applications cited herein are incorporated herein by reference in their entirety.

### Industrial Applicability

The compounds of the present invention have a strong inhibitory effect of platelet aggregation and are effective as therapeutic agents and prophylactics for diseases in which thrombus is involved.

## Claims

1. A benzomorpholine derivative or pharmaceutically acceptable salt thereof represented by formula I, 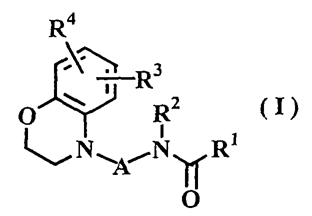 wherein
A is C₂₋₄ alkylene, C₂₋₄ alkenylene, or C₂₋₄ alkynylene,
R¹ is:
(1) unsubstituted aryl or heteroaryl, or aryl or heteroaryl substituted with one or a plurality of substituents independently selected from the following group,
a) C₁₋₅ alkyl, b) C₁₋₅ alkoxy c) C₃₋₈ cycloalkyl, d) C₁₋₅ haloalkyl, e) phenyl, f) phenoxy, g) hydroxyl, h) C₁₋₅ hydroxyalkyl, i) C₁₋₅ haloalkyloxy, j) mercapto, k) C₁₋₅ alkylthio, 1) C₁₋₅ haloalkylthio, m) halogen, n) cyano, o) nitro, p) amino, q) C₁₋₅ alkylamino, r) C₂₋₁₀ dialkylamino, s) acyl, t) carboxyl, u) C₂₋₆ alkyloxycarbonyl, v) mesyl, w) trifluoromethanesulfonyl, and x) tosyl; or
(2) unsubstituted C₁₋₅ alkyl, C₃₋₈ cycloalkyl, C₂₋₁₀ alkenyl, C₄₋₁₀ cycloalkenyl, or C₂₋₁₀ alkynyl, or C₁₋₅ alkyl, C₃₋₈ cycloalkyl, C₂₋₁₀ alkenyl, C₄₋₁₀ cycloalkenyl, or C₂₋₁₀ alkynyl substituted with one or a plurality of substituents independently selected from the following group,
a) phenyl, b) hydroxyl, c) C₁₋₅ alkyl, d) C₃₋₈ cycloalkyl, e) C₁₋₅ haloalkyl, and f) halogen;
R² is unsubstituted aryl or heteroaryl, or aryl or heteroaryl substituted with one or a plurality of substituents independently selected from the following group,
a) C₁₋₅ alkyl, b) C₁₋₅ alkoxy, c) C₃₋₈ cycloalkyl, d) C₁₋₅ haloalkyl, e) phenyl, f) phenoxy, g) hydroxyl, h) C₁₋₅ hydroxyalkyl, i) C₁₋₅ haloalkyloxy, j) mercapto, k) C₁₋₅ alkylthio, 1) C₁₋₅ haloalkylthio, m) halogen, n) cyano, o) nitro, p) amino, q) C₁₋₅ alkylamino, r) C₂₋₁₀ dialkylamino, s) acyl, t) carboxyl, u) C₂₋₆ alkyloxycarbonyl, v) mesyl, w) trifluoromethanesulfonyl, and x) tosyl;
R³ is hydrogen, halogen, C₁₋₅ alkyl, or C₁₋₅ alkoxy; R⁴ is -X- (CH₂)n -COOR⁵, and X is -O-, -S-, or -CH₂-; R⁵ is hydrogen or C₁₋₅ alkyl; and n is an integer that is 1, 2, or 3.

2. The benzomorpholine derivative or pharmaceutically acceptable salt thereof according to claim 1 represented by general formula (II), 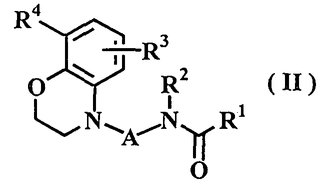 wherein A, R¹, R², R³, and R⁴ are as defined in claim 1.

3. The benzomorpholine derivative or pharmaceutically acceptable salt thereof according to claim 1, wherein A is ethylene.

4. The benzomorpholine derivative or pharmaceutically acceptable salt thereof according to claim 1, wherein R¹ is unsubstituted aryl or heteroaryl, or aryl or heteroaryl substituted with one or a plurality of substituents which are as defined in claim 1.

5. The benzomorpholine derivative or pharmaceutically acceptable salt thereof according to claim 4, wherein R¹ is unsubstituted phenyl, furyl, thienyl, or pyridyl, or phenyl, furyl, thienyl, or pyridyl substituted with one or a plurality of substituents which are as defined in claim 1.

6. The benzomorpholine derivative or pharmaceutically acceptable salt thereof according to claim 5, wherein R¹ is unsubstituted phenyl, furyl, thienyl, or pyridyl, or phenyl, furyl, thienyl, or pyridyl substituted with one or a plurality of substituents independently selected from the following group,
a) C₁₋₅ alkyl, b) C₁₋₅ alkoxy, c) C₁₋₅ haloalkyl, d) hydroxyl, e) C₁₋₅ haloalkyloxy, f) C₁₋₅ alkylthio, g) C₁₋₅ haloalkylthio, h) halogen, i) cyano, j) C₂₋₁₀ dialkylamino, k) acetyl, 1) C₂₋₆ alkyloxycarbonyl, m) mesyl, n) trifluoromethanesulfonyl, and o) tosyl.

7. The benzomorpholine derivative or pharmaceutically acceptable salt thereof according to claim 6, wherein R¹ is unsubstituted phenyl, furyl, thienyl, or pyridyl or phenyl, furyl, thienyl, or pyridyl substituted with one or a plurality of substituents independently selected from the following group,
a) C₁₋₅ alkyl, b) C₁₋₅ alkoxy, c) C₁₋₅ haloalkyl, d) hydroxyl, h) halogen, and i) cyano.

8. The benzomorpholine derivative or pharmaceutically acceptable salt thereof according to claim 1, wherein R² is unsubstituted phenyl or pyridyl, or phenyl or pyridyl substituted with one or a plurality of substituents which are as defined in claim 1.

9. The benzomorpholine derivative or pharmaceutically acceptable salt thereof according to claim 8, wherein R² is unsubstituted phenyl or pyridyl, or phenyl or pyridyl substituted with one or a plurality of substituents independently selected from the following group,
a) C₁₋₅ alkyl, b) C₁₋₅ alkoxy, c) C₁₋₅ haloalkyl, d) hydroxyl, e) C₁₋₅ haloalkyloxy, f) C₁₋₅ alkylthio, g) C₁₋₅ haloalkylthio, h) halogen, i) cyano, j) amino, k) C₂₋₁₀ dialkylamino, 1) acyl, m) C₂₋₆ alkyloxycarbonyl, n) mesyl, o) trifluoromethanesulfonyl, and p) tosyl.

10. The benzomorpholine derivative or pharmaceutically acceptable salt thereof according to claim 9, wherein R² is unsubstituted phenyl or pyridyl, or phenyl or pyridyl substituted with one or a plurality of substituents independently selected from the following group,
a) C₁₋₅ alkyl, b) C₁₋₅ alkoxy, c) C₁₋₅ haloalkyl, d) C₁₋₅ haloalkyloxy, e) C₁₋₅ alkylthio, f) halogen, and g) C₂₋₁₀ dialkylamino.

11. The benzomorpholine derivative or pharmaceutically acceptable salt thereof according to claim 1, wherein X is -O-.

12. A drug comprising the benzomorpholine derivative according to claim 1 as an active ingredient.

13. A platelet aggregation inhibitor or prophylactic comprising the benzomorpholine derivative according to claim 1 as an active ingredient.

14. The platelet aggregation inhibitor or prophylactic according to claim 13, which is used for treating or preventing thrombosis or diseases accompanying thrombus.

15. The platelet aggregation inhibitor or prophylactic according to claim 14, wherein the thrombosis is thrombosis in coronary arteries, cerebral arteries, peripheral arteries, or peripheral veins.

16. The platelet aggregation inhibitor or prophylactic according to claim 14, wherein the disease accompanying thrombus is myocardial infarction, unstable angina, cerebral infarction, transient ischemic attack, or chronic arterial occlusion.
